# EUROPEAN PATENT APPLICATION

(11) **EP 4 011 365 A1**
(43) Date of publication of application: **15.06.2022**
(21) Application number: 20212892.2
(22) Date of filing: 09.12.2020
(51) Int. Cl.: A61K 9/51, A61K 31/00, A61K 47/69, A61K 31/704

(54) **MOF NANOPARTICLES**

(71) Applicant: Cambridge Enterprise, Ltd., Cambridge Cambridgeshire CB2 1TN (GB)
(72) Inventor: The designation of the inventor has not yet been filed
(74) Representative: Patent Boutique LLP

(57) **Abstract**

The present application relates to metal-organic framework (MOF) nanoparticles, in particular, coated MOF nanoparticles, methods of manufacturing said coated MOF nanoparticles, and uses of said coated MOF nanoparticles.

## Description

### Field of the Invention

The present invention relates to metal-organic framework (MOF) nanoparticles, in particular, coated MOF nanoparticles, methods of manufacturing said coated MOF nanoparticles, and uses of said coated MOF nanoparticles.

### Background of the Invention

Metal-organic framework (MOF) nanoparticles (nanoMOFs) are a relatively new family of porous, crystalline hybrid materials that have shown potential in a variety of applications such as gas storage and separation, sensing, and catalysis. In light of their tuneable structures and permanent porosities, both organic ligands and inorganic nodes (metal ions) provide platforms for incorporating functionality into their internal and external surface. External surface functionalisation of nanoMOFs has been studied in biological systems for imaging and therapeutic applications via post-synthetic modifications.

While nanoMOFs have shown promise, known surface-functionalised nanoMOFs have their limitations. For instance, the inventors have found that when known surface-functionalised nanoMOFs are dried they cannot be easily re-dispersed. As a result, generally, they have had to be kept in suspension before use. This has made them unattractive to fields such as drug delivery, as known nanoparticle MOFs in solution may provide a limited shelf-life due to aggregation and/or premature or uncontrolled drug release and/or deterioration of the active. Moreover, there is a long-felt and ongoing need for drug delivery systems with tuneable release profiles.

The present invention addresses these, and other issues associated with known MOF nanoparticles.

### Summary of the Invention

Accordingly, in a first aspect, the present invention provides a composition comprising one or more polymer-coated metal-organic framework (MOF) nanoparticles.

Each nanoparticle comprises a MOF comprising a plurality of metal ions (inorganic nodes) and a plurality of organic ligands. Preferably, the plurality of metal ions may (as a plurality) consist essentially of (that is to say substantially all of the metal ions within the MOF nanoparticle are the same), or consist of, ions of a metal selected from the group consisting of: zirconium, iron, zinc, hafnium, magnesium and potassium; more preferably the plurality of metal ions may consist essentially of ions, or consist of ions, of a metal selected from the group consisting of: zirconium and iron; most preferably the plurality of metal ions may consist essentially of, or consist of, ions of zirconium.

The polymer is attached to one or more metal ions of each nanoparticle by phosphate-metal coordination. Preferably the polymer includes a phosphate group, more preferably the polymer is phosphate-terminated. Preferably, each polymer has a single terminal phosphate group. Typically, the polymer comprises a polymer (base polymer) which has been phosphate functionalised. Preferably, the base polymer is hydrophilic. Preferably, the polymer (and/or base polymer) is selected from the group consisting of polyethylene glycol, hyaluronic acid, heparin, chitosan, polyvinyl alcohol, polyglutamic acid, and derivatives and copolymers thereof. More preferably, the polymer comprises polyethylene glycol or a derivative thereof.

In embodiments, when the polymer comprises polyethylene glycol or a derivative thereof, preferably the polyethylene glycol is linear polyethylene glycol or a derivative thereof. Preferably the polyethylene glycol is predominantly linear polyethylene glycol or a derivative thereof, more preferably the polymer may consist essentially of, or consists of, linear polyethylene glycol or a derivative thereof. Preferably, the polymer is not or is free from branched polyethylene glycol or branched derivatives thereof.

Preferably, the polyethylene glycol or derivative thereof comprises from about 40 to about 250 repeating units.

Preferably the polymer comprises, or consists essentially of, a phosphate-terminated linear alkoxy polyethylene glycol (PEG), wherein the alkoxy group is preferably a C₁ to C₅ alkoxy, more preferably a C₁ to C₃ alkoxy. Even more preferably, the polymer comprises, or consists essentially of, a phosphate-terminated linear methoxy polyethylene glycol (mPEG).

Most preferably, the polymer is a phosphate-terminated mPEG according to the structure A.

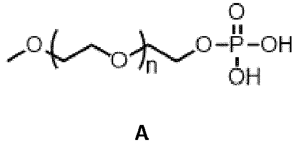

Preferably n is from about 40 to about 250.

Typically, the mPEG may have a molecular weight of from about 2000 g/mol to about 10000 g/mol (mPEG2000 to mPEG 10000), preferably from about 3000 g/mol to about 9000 g/mol (mPEG3000 to mPEG9000), from about 4000 g/mol to about 8000 g/mol (mPEG4000 to mPEG8000), from about 5000 g/mol to about 7000 g/mol (mPEG5000 to mPEG7000), from about 5000 g/mol to about 6000 g/mol (mPEG5000 to mPEG6000). Preferably, the mPEG has a molecular weight of about 5000 g/mol (mPEG5000).

The polymer is attached to one or more metal ions of each MOF nanoparticle, preferably substantially all of the MOF nanoparticles. Preferably, the polymer is attached to metal ions located at an external surface of each MOF nanoparticle. Preferably, an external surface of each MOF nanoparticle is substantially coated in the polymer. The external surface refers to the outside surface, not for instance an internal surface, such as that of a pore.

The particle size of the one or more polymer-coated MOF nanoparticles may be from about 25 nm to about 450 nm, more preferably from about 100 nm to about 250 nm, most preferably from about 100 nm to 200 nm. Preferably, an active ingredient may be encapsulated within and/or located on the one or more polymer-coated MOF nanoparticles. Preferably, the active ingredient may be selected from the group consisting of: pharmaceutically active ingredients, pesticides, insecticides, and dyes.

Compositions according to the first aspect of the invention are particularly advantageous as they may have improved stability, a tuneable release profile, facilitate lyophilisation and/or dispersion of the composition in solution, such that the one or more polymer-coated MOF nanoparticles do not agglomerate significantly.

In a second aspect, the present invention provides a dry powder pharmaceutical composition comprising one or more lyophilised polymer-coated metal-organic framework (MOF) nanoparticles. Each nanoparticle comprises a MOF comprising a plurality of metal ions (inorganic nodes) and a plurality of organic ligands.

As in the previous aspect, preferably, the plurality of metal ions may consist essentially of, or consist of, ions of a metal selected from the group consisting of: zirconium, iron, zinc, and hafnium; more preferably the plurality of metal ions may consist essentially of, or consist of, ions of a metal selected from the group consisting of: zirconium and iron; most preferably the plurality of metal ions may consist essentially of, or consist of, ions of zirconium.

The polymer may be attached to one or more metal ions of each nanoparticle by phosphate-metal coordination. Preferably the polymer includes a phosphate group, more preferably the polymer is phosphate-terminated. Preferably, each polymer has a single terminal phosphate group. Typically, the polymer comprises a polymer (base polymer) which has been phosphate functionalised. Preferably, the base polymer is hydrophilic.

Preferably, the polymer (and/or base polymer) is selected from the group consisting of polyethylene glycol, hyaluronic acid, heparin, chitosan, polyvinyl alcohol, polyglutamic acid, and derivatives and copolymers thereof. More preferably, the polymer comprises polyethylene glycol or a derivative thereof.

In embodiments, when the polymer comprises polyethylene glycol or a derivative thereof, preferably the polyethylene glycol is linear polyethylene glycol or a derivative thereof. Preferably the polyethylene glycol is predominantly linear polyethylene glycol or a derivative thereof, more preferably the polymer may consist essentially of, or consists of, linear polyethylene glycol or a derivative thereof. Preferably, the polymer is not or is free from branched polyethylene glycol or derivatives thereof.

Preferably, the polyethylene glycol or derivative thereof comprises from about 40 to about 250 repeating units.

Preferably the polymer comprises, or consists essentially of, a phosphate-terminated linear alkoxy polyethylene glycol (PEG), wherein the alkoxy group is preferably a C₁ to C₅ alkoxy, more preferably a C₁ to C₃ alkoxy. Even more preferably, the polymer comprises, or consists essentially of, a phosphate-terminated linear methoxy polyethylene glycol (mPEG).

More preferably, the polymer is a phosphate-terminated mPEG according to the structure A.

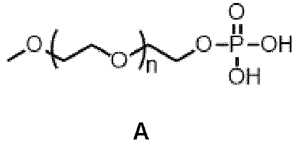

Preferably n is from about 40 to about 250.

Typically, the mPEG may have a molecular weight of from about 2000 g/mol to about 10000 g/mol (mPEG2000 to mPEG 10000), preferably from about 3000 g/mol to about 9000 g/mol (mPEG3000 to mPEG9000), from about 4000 g/mol to about 8000 g/mol (mPEG4000 to mPEG8000), from about 5000 g/mol to about 7000 g/mol (mPEG5000 to mPEG7000), from about 5000 g/mol to about 6000 g/mol (mPEG5000 to mPEG6000). Preferably, the mPEG has a molecular weight of about 5000 g/mol (mPEG5000).

Preferably, the particle size of the one or more lyophilised polymer-coated MOF nanoparticles of the second aspect may be from about 25 nm to about 450 nm, more preferably from about 100 nm to about 250 nm, most preferably from about 100 nm to about 200 nm.

Polymer is attached to one or more metal ions of each MOF nanoparticle, preferably substantially all of the MOF nanoparticles. Preferably, the polymer is attached to metal ions located at the external surface of each MOF nanoparticle. Preferably, the external surface of each MOF nanoparticle is substantially coated in the polymer.

Preferably, the polymer may be attached to one or more metal ions of each nanoparticle via metal coordination, more preferably by phosphate-metal coordination.

At least one active pharmaceutical ingredient is located within and/or on one or more of the lyophilised polymer-coated MOF nanoparticles. Preferably the one active pharmaceutical ingredient is located within and/or on the one or more MOF nanoparticles before they are polymer-coated, preferably by diffusion from a suspension comprising the active pharmaceutical ingredient, typically in solution, and the uncoated MOF nanoparticles in suspension.

Dry powder pharmaceutical compositions according to the second aspect of the invention are particularly advantageous as such compositions may have improved stability and/or these dry powders may be easily redispersed in an aqueous medium, showing substantially no agglomeration of the redispersed lyophilised polymer-coated pharmaceutical-containing MOF nanoparticles. Moreover, the release of active pharmaceutical ingredient from the polymer-coated MOF nanoparticles may be tuned by varying amount and/or species of the coating polymer.

Preferably, the dry powder pharmaceutical composition is provided for use in a vial, preferably wherein the vial has a volume of from about 5 ml to about 25 ml. Preferably, during manufacture the dry powder pharmaceutical composition is lyophilised in said vial. Typically, in use, the dry powder pharmaceutical composition is redispersed in a liquid vehicle in said vial.

The polymer-coated MOF nanoparticles may contain a pharmaceutically active ingredient. The pharmaceutically active ingredient may be present at an amount of at least about 0.5 % , at least about 1 %, at least about 2 % or at least about 4 % by weight of the total weight of the polymer-coated pharmaceutical-containing MOF nanoparticles. In embodiments, the component is present at an amount of at most about 10 %, at most about 15 %, at most about 20 %, at most about 25 %, at most about 35 %, at most about 50 %, or at most about 65 % by weight of the total weight of the polymer-coated pharmaceutical-containing MOF nanoparticles. In one embodiment, the component is present at an amount of at most 30 % by weight of the total weight of the polymer-coated pharmaceutical-containing MOF nanoparticles. Ranges of from about 0.5 % to about 60 %, or from about 10 % to about 50 % by weight of the total weight of the polymer-coated pharmaceutical-containing MOF nanoparticles are preferred.

Alternatively, the active pharmaceutical ingredient may be present at an amount of at most about 0.5 %, at most about 1 %, at most about 2 % or at most about 4 % by weight of the total weight of the polymer-coated pharmaceutical-containing MOF nanoparticles The active pharmaceutical ingredient may be present at an amount of at least about 0.001 %, at least about 0.005 %, at least about 0.01 %, at least about 0.05 % or at least about 0.1 % by weight of the total weight of the polymer-coated pharmaceutical-containing MOF nanoparticles.

In embodiments, the metal-organic framework of the MOF nanoparticles may be at least partially amorphous, preferably substantially amorphous. Preferably, the MOF nanoparticles are amorphised once the pharmaceutically active ingredient is within the MOF nanoparticle, preferably before polymer coating of the MOF nanoparticles is performed, preferably before lyophilisation thereof also. Suitable methods for amorphising metal-organic frameworks are disclosed below and in WO2016/207397 which is incorporated herein by reference, e.g. ball milling.

The amorphization process is intended to destroy the long-range order that is present within a crystalline metal-organic framework. The amorphous metal-organic framework has a highly disordered framework structure and lacks long-range order. However, the amorphous framework retains basic metal-ligand connectivity that is also present in the crystalline metal-organic framework. The amorphization process may be said to result in the structural collapse of the crystalline metal-organic framework. This structural collapse may, in use, slow or augment the release of the pharmaceutically active ingredient from the MOF nanoparticles.

The change in the metal-organic framework from a crystalline to an amorphous structure may be established by X-ray diffraction methods, and powder X-ray diffraction methods in particular. Typically the crystalline metal-organic framework possesses distinct Bragg reflections in the PXRD pattern. In the PXRD pattern for the amorphised metal-organic framework, these Bragg reflections are lost.

Advantageously, the inventors have found that amorphisation complements polymer-coating of the MOF nanoparticles to provide still further tuneability to the active pharmaceutical release profile, typically to an extent not achievable by either technique taken alone.

In a third aspect, the present invention provides a pharmaceutical composition comprising the dry powder pharmaceutical composition according to the second aspect mixed into a liquid (i.e. a vehicle). It will be understood that a vehicle is a carrier composed of one or more excipients for active pharmaceutical ingredient(s) in a liquid preparation. The pharmaceutical composition of the third aspect may be provided as a solution, a suspension or a colloid, preferably an aqueous solution, an aqueous suspension or an aqueous colloid. The aqueous phase may be water, bovine serum albumin, phosphate-buffered saline, plasma, Dulbecco's Modified Eagle Medium (DMEM), Eagle's Minimum Essential Medium (EMEM), Gibco Roswell Park Memorial Institute (RPMI) 1640 medium, McCoy's 5A medium, and/or Hanks' Balanced Salt Solution (HBSS), preferably the aqueous phase is water. Typically, the dry powder pharmaceutical composition is mixed with the liquid vehicle immediately before use.

Preferably, the pharmaceutical composition further comprises a pharmaceutically acceptable excipient, preferably wherein the excipient is selected from the group consisting of buffers, stabilisers, preservatives, colouring agents, flavouring agents, cosolvents, and combinations thereof.

In some embodiments, the pharmaceutically acceptable excipient may be provided in the liquid vehicle with which the pharmaceutical active ingredient-containing polymer-coated MOF nanoparticles are mixed. Additionally, or alternatively, the pharmaceutically acceptable excipient may be premixed with the dry powder composition pharmaceutical active ingredient-containing polymer-coated MOF nanoparticles before they are combined with the liquid vehicle.

Pharmaceutical compositions according to the third aspect of the invention are particularly advantageous because the redispersed lyophilised polymer-coated MOF nanoparticles generally do not agglomerate. Additionally, or alternatively, the active pharmaceutical ingredient may remain substantially within and/or on the polymer-coated MOF nanoparticles while in the liquid vehicle before administration. Moreover, the release of active pharmaceutical ingredient from the polymer-coated MOF nanoparticles may be tuned. This may be achieved, for instance, by varying the amount of polymer coating on an external surface of each MOF nanoparticle, which may, in turn, be varied by increasing or decreasing the coating period. Additionally, or alternatively, the species of polymer(s) used for coating the nanoparticles may be varied. Additionally, or alternatively, the metal-organic frameworks of nanoparticle MOFs may be amorphized (fully or partially) as discussed herein.

In all aspects, the period of time for which polymer coating occurs (i.e. the coating period) may be determined by the properties desired. Typically, the coating time is sufficient for the metal sites on the external surface of the MOF nanoparticles to be substantially saturated (e.g. substantially coated), preferably with limited or substantially no reduction of the MOF nanoparticles' porosity. Typically, the coating time is less than about three hours, preferably less than about two hours, preferably from about 1 minute to about 180 minutes, preferably from about 30 minutes to about 150 minutes, preferably from about 5 minutes to about 60 minutes.

Preferably the N₂ adsorption uptake of the polymer-coated nanoparticle MOF is greater than about 80 % of the ideal N₂ uptake, preferably greater than about 90 %.

Preferably, the polymer coating makes up less than about 40 % by weight of the polymer-coated nanoparticle MOF, preferably less than about 30 % by weight, more preferably from about 10 % by weight to about 40 % by weight, preferably from about 20 % by weight to about 35 % by weight of the polymer-coated nanoparticle MOF.

In a fourth aspect, the present invention provides a method of producing a dry powder composition comprising one or more polymer-coated metal-organic framework (MOF) nanoparticles. Each nanoparticle comprises a MOF comprising a plurality of metal ions (inorganic nodes) and a plurality of organic ligands.

As in the previous aspects, preferably, the plurality of metal ions may consist essentially of, or consist of, ions of a metal selected from the group consisting of zirconium, iron, zinc, hafnium, magnesium and potassium, more preferably the plurality of metal ions may consist essentially of, or consist of, ions of a metal selected from the group consisting of zirconium and iron; most preferably the plurality of metal ions may consist essentially of, or consist of ions of zirconium.

Polymer is attached to one or more metal ions. Preferably the polymer includes a phosphate group, more preferably the polymer is phosphate-terminated, preferably the polymer has a single terminal phosphate group. Typically, the polymer comprises a base polymer which has been phosphate functionalised. Preferably, the base polymer is hydrophilic.

Preferably, the polymer may be selected from the group consisting of polyethylene glycol, hyaluronic acid, heparin, chitosan, polyvinyl alcohol, polyglutamic acid, and derivatives and copolymers thereof.

In embodiments, when the polymer comprises polyethylene glycol or a derivative thereof, preferably the polyethylene glycol is linear polyethylene glycol or a derivative thereof. Preferably the polyethylene glycol is predominantly linear polyethylene glycol or a derivative thereof, more preferably the polymer may consist essentially of, or consists of, linear polyethylene glycol or a derivative thereof. Preferably, the polymer is not a branched polyethylene glycol and/or is substantially free from branched polyethylene glycol, or derivatives thereof.

Preferably, the polyethylene glycol or derivative thereof comprises from about 40 to about 250 repeating units.

Preferably the polymer comprises, or consists essentially of, a phosphate-terminated linear alkoxy polyethylene glycol (PEG), wherein the alkoxy group is preferably a C₁ to C₅ alkoxy, more preferably a C₁ to C₃ alkoxy. Even more preferably, the polymer comprises, or consists essentially of, a phosphate-terminated linear methoxy polyethylene glycol (mPEG).

Most preferably, the polymer is a phosphate-terminated mPEG according to the structure A.

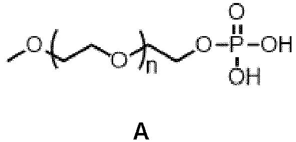

Preferably n is from about 40 to about 250.

Typically, the mPEG (such as that of structure A) may have a molecular weight of from about 2000 g/mol to about 10000 g/mol (mPEG2000 to mPEG10000), preferably from about 3000 g/mol to about 9000 g/mol (mPEG3000 to mPEG9000), from about 4000 g/mol to about 8000 g/mol (mPEG4000 to mPEG8000), from about 5000 g/mol to about 7000 g/mol (mPEG5000 to mPEG7000), from about 5000 g/mol to about 6000 g/mol (mPEG5000 to mPEG6000). Preferably, the mPEG has a molecular weight of about 5000 g/mol (mPEG5000).

The size of the one or more polymer-coated MOF nanoparticles may be from about 25 nm to about 450 nm, preferably from about 100 nm to about 250 nm, most preferably from about 100 nm to about 200 nm.

The method of the fourth aspect comprises the following steps: mixing a plurality of metal ions (inorganic nodes) and a plurality of organic ligands to form a suspension of one or more uncoated MOF nanoparticles; mixing a suspension of the one or more uncoated MOF nanoparticles with the polymer and allowing polymer to attach to one or more metal ions of the one or more uncoated MOF nanoparticles to provide a suspension of the one or more polymer-coated MOF nanoparticles; and drying the one or more polymer-coated MOF nanoparticles by lyophilisation (i.e. freeze drying).

Optionally, the methods of the invention may include a condensing step in which the polymer-coated MOF nanoparticle solution is made more concentrated (i.e. a concentrated slurry). Typically, by increasing the concentration of the polymer-coated MOF nanoparticle suspension by removing solvent, for instance by centrifugation, evaporation or other suitable methods.

Preferably, the suspension of the one or more polymer-coated MOF nanoparticles has a solids content, by reference to the total weight of the suspension prior to lyophilisation, of from about 1% to about 25%, preferably from about 2% to about 10%. Preferably, the suspension is an aqueous suspension.

Preferably, an active ingredient is located within (i.e. encapsulated within) and/or on the one or more polymer-coated MOF nanoparticles, preferably the active ingredient is encapsulated within and/or located on the uncoated MOF nanoparticles prior to the step of mixing a suspension of the one or more uncoated MOF nanoparticles with the polymer and allowing polymer to attach to one or more metal ions of the one or more uncoated MOF nanoparticles to provide a suspension of the one or more polymer-coated MOF nanoparticles.

Preferably, an active ingredient is encapsulated within and/or located on the one or more polymer-coated MOF nanoparticles by mixing an active ingredient with a suspension of the one or more uncoated MOF nanoparticles. Preferably, the active ingredient may be selected from the group consisting of: pharmaceutically active ingredients, pesticides, insecticides, and dyes.

The method according to the fourth aspect of the invention is particularly advantageous as the lyophilisation step of this method provides a dry powder composition that may be subsequently redispersed in an aqueous medium with reduced agglomeration of the one or more polymer-coated MOF nanoparticles. This cannot be achieved by ambient drying. Advantageously, such dry compositions may have better shelf-lives than corresponding aqueous suspensions.

Preferably, the dry powder pharmaceutical composition is provided for use in a vial, preferably wherein the vial has a volume of from about 5 ml to about 25 ml. Preferably during manufacture, the dry powder pharmaceutical composition formed by lyophilising a liquid slurry containing the polymer-coated pharmaceutical-containing MOF nanoparticles in one or more of the said vial, preferably a plurality of vials each containing a liquid slurry containing the polymer-coated pharmaceutical-containing MOF nanoparticles undergo lyophilisation simultaneously.

Preferably, in use, the dry powder pharmaceutical composition is redispersed in a liquid vehicle in the said vial.

In a fifth aspect, the invention provides a method of producing a suspension of polymer-coated MOF nanoparticles, the method comprising the step of taking the dry powder composition produced according to the fourth aspect and resuspending the composition in a liquid.

Preferably, the liquid is aqueous, preferably an aqueous liquid selected from the group consisting of: water, bovine serum albumin, phosphate-buffered saline, plasma, Dulbecco's Modified Eagle Medium (DMEM), Eagle's Minimum Essential Medium (EMEM), Gibco Roswell Park Memorial Institute (RPMI) 1640 medium, McCoy's 5A medium, and Hanks' Balanced Salt Solution (HBSS). Preferably, the aqueous liquid is water.

The method according to the fifth aspect of the invention is particularly advantageous as the method may allow the polymer-coated MOF nanoparticles to be dispersed in a liquid such that the one or more polymer-coated MOF nanoparticles may not agglomerate.

In a sixth aspect, the present invention provides a method of producing a composition comprising one or more polymer-coated active ingredient-containing metal-organic framework (MOF) nanoparticles. Each nanoparticle comprising a MOF comprising a plurality of metal ions (inorganic nodes) and a plurality of organic ligands.

Preferably, the plurality of metal ions may consist essentially of, or consist of, ions of a metal selected from the group consisting of: zirconium, iron, zinc, hafnium, magnesium or potassium; more preferably the plurality of metal ions may consist essentially of, or consist of, ions of a metal selected from the group consisting of: zirconium and iron; most preferably the plurality of metal ions may consist essentially, or consist of, ions of zirconium.

Preferably, at least one active ingredient may be selected from the group consisting of: pharmaceutically active ingredients, pesticides, insecticides, and dyes.

The particle size of the one or more polymer-coated active ingredient-containing MOF nanoparticles according to the sixth aspect may be from about 25 nm to about 450 nm, preferably from about 100 nm to about 250 nm, most preferably from about 100 to about 200 nm.

The polymer is attached to one or more metal ions by metal coordination, preferably phosphate-metal coordination. Preferably the polymer includes a phosphate group, more preferably the polymer is phosphate-terminated. Typically, the polymer comprises a base polymer which has been phosphate functionalised.

Preferably, the polymer (or base polymer) may be selected from the group consisting of polyethylene glycol, hyaluronic acid, heparin, chitosan, polyvinyl alcohol, polyglutamic acid, and derivatives and copolymers thereof.

In embodiments, when the polymer comprises polyethylene glycol or a derivative thereof, preferably the polyethylene glycol is linear polyethylene glycol or a derivative thereof. Preferably the polyethylene glycol is predominantly linear polyethylene glycol or a derivative thereof, more preferably the polymer may consist essentially of, or consists of, linear polyethylene glycol or a derivative thereof. Preferably, the polymer is not a branched polyethylene glycol and/or is substantially free from branched polyethylene glycol.

Preferably, the polyethylene glycol or derivative thereof comprises from about 40 to about 250 repeating units.

Preferably the polymer comprises, or consists essentially of, a phosphate-terminated linear alkoxy polyethylene glycol (PEG), wherein the alkoxy group is preferably a C₁ to C₅ alkoxy, more preferably a C₁ to C₃ alkoxy. Even more preferably, the polymer comprises, or consists essentially of, a phosphate-terminated linear methoxy polyethylene glycol (mPEG).

Preferably, the polymer is a phosphate-terminated mPEG according to structure A.

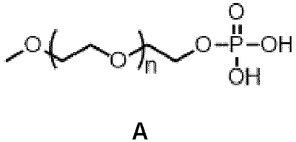

Preferably n is from about 40 to about 250.

Typically, the mPEG (such as the mPEG of structure A) may have a molecular weight of from about 2000 g/mol to about 10000 g/mol (e.g. mPEG2000 to mPEG10000), preferably from about 3000 g/mol to about 9000 g/mol (e.g. mPEG3000 to mPEG9000), from about 4000 g/mol to about 8000 g/mol (e.g. mPEG4000 to mPEG8000), from about 5000 g/mol to about 7000 g/mol (e.g. mPEG5000 to mPEG7000), from about 5000 g/mol to about 6000 g/mol (e.g. mPEG5000 to mPEG6000). Preferably, the mPEG has a molecular weight of about 5000 g/mol (e.g. mPEG5000).

Preferably, the polymer is attached to metal ions located at an external surface of each MOF nanoparticle. Preferably, an external surface of each MOF nanoparticle is substantially coated in the polymer.

The method of the sixth aspect comprises the steps of: i) mixing a plurality of metal ions (inorganic nodes) and a plurality of organic ligands to form a suspension of one or more uncoated MOF nanoparticles; ii) encapsulating an active ingredient within one or more of the uncoated MOF nanoparticles to provide a suspension of one or more active ingredient-containing, uncoated MOF nanoparticles; iii) subsequently mixing a suspension of the one or more active ingredient-containing, uncoated MOF nanoparticles with a phosphate-functionalised polymer and allowing the phosphate-functionalised polymer to coordinate to one or more metal ions of the one or more active ingredient-containing, uncoated MOF nanoparticles to provide a suspension of one or more active ingredient-containing polymer-coated MOF nanoparticles; iv) optionally drying the one or more polymer-coated active ingredient-containing MOF nanoparticles by lyophilisation; and v) optionally, subsequently redispersing the lyophilised polymer-coated active ingredient-containing MOF nanoparticles in an aqueous medium.

Preferably, the aqueous redispersion medium is selected from the group consisting of: water, bovine serum albumin, phosphate-buffered saline, plasma, Dulbecco's Modified Eagle Medium (DMEM), Eagle's Minimum Essential Medium (EMEM), Gibco Roswell Park Memorial Institute (RPMI) 1640 medium, McCoy's 5A medium, and Hanks' Balanced Salt Solution (HBSS). Preferably, the aqueous redispersion medium is water.

Preferably, the suspension of the one or more polymer-coated MOF nanoparticles has a solids content of from about 1% to about 25%, preferably from about 2% to about 10%, of the total weight of the suspension prior to lyophilisation.

Typically, the coordination step (iii) is performed for a period of time (the coating time) sufficient for the metal sites on the external surface of the MOF nanoparticles to be substantially saturated, preferably with polymer with limited or substantially no reduction of the MOF nanoparticles' porosity. Typically, step iii) lasts less than about three hours, preferably less than about two hours, preferably from about 1 minute to about 180 minutes, preferably from about 30 minutes to about 150 minutes, preferably from about 5 minutes to about 60 minutes.

Preferably the N₂ sorption uptake of the polymer-coated nanoparticle MOF is greater than about 80 % of the ideal N₂ uptake, preferably greater than about 90 %.

Preferably, the polymer coating makes up less than about 40 % by weight of the polymer-coated nanoparticle MOF, preferably less than about 30 % by weight, more preferably from about 10 % by weight to about 40 % by weight, preferably from about 20 % by weight to about 35 % by weight of the polymer-coated nanoparticle MOF.

The method according to the sixth aspect of the invention is particularly advantageous as the method produces a stable suspension of one or more polymer-coated active ingredient-containing MOF nanoparticles, which may have improved stability during storage. Additionally, if the one or more polymer-coated active ingredient-containing MOF nanoparticles are prepared by lyophilisation the composition may be stable during storage and subsequently, on redispersion, provide a suspension of the one or more polymer-coated active ingredient-containing MOF nanoparticles with reduced aggregation.

In a seventh aspect, the present invention provides method of producing a composition comprising a one or more methoxy polyethylene glycol (mPEG)-coated metal-organic framework (MOF) nanoparticles, each nanoparticle comprising a MOF comprising a plurality of metal ions (inorganic nodes) and a plurality of organic ligands.

Preferably, the plurality of metal ions may consist essentially of, or consist of, ions of a metal selected from the group consisting of: zirconium, iron, zinc, hafnium, magnesium and potassium; more preferably the plurality of metal ions may consist essentially of, or consist of, ions of a metal selected from the group consisting of: zirconium and iron; most preferably the plurality of metal ions may consist essentially of, or consist of, ions of zirconium.

The particle size of the one or more methoxy mPEG-coated MOF nanoparticles may be from about 25 nm to about 450 nm, preferably from about 100 nm to about 250 nm, most preferably from about 100 nm to about 200 nm. mPEG is attached to one or more metal ions by metal coordination, preferably phosphate-metal coordination.

Preferably, the mPEG is a phosphate-terminated mPEG according to structure A.

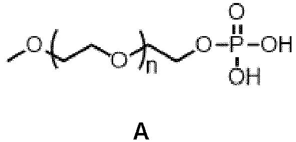

Preferably n is from about 40 to about 250.

Typically, the mPEG may have a molecular weight of from about 2000 g/mol to about 10000 g/mol (mPEG2000 to mPEG 10000), preferably from about 3000 g/mol to about 9000 g/mol (mPEG3000 to mPEG9000), from about 4000 g/mol to about 8000 g/mol (mPEG4000 to mPEG8000), from about 5000 g/mol to about 7000 g/mol (mPEG5000 to mPEG7000), from about 5000 g/mol to about 6000 g/mol (mPEG5000 to mPEG6000). Preferably, the mPEG has a molecular weight of about 5000 g/mol (mPEG5000).

The method of the seventh aspect comprises the steps of: mixing a plurality of metal ions and a plurality of organic ligands to form a suspension of one or more uncoated MOF nanoparticles; mixing a suspension of the one or more uncoated MOF nanoparticles with a solution of mPEG-PO₃ and allowing mPEG-PO₃ to coordinate to one or more metal ions of the one or more uncoated MOF nanoparticles to provide a suspension of the one or more mPEG-coated MOF nanoparticles; optionally, subsequently drying the one or more mPEG-coated MOF nanoparticles by lyophilisation; and optionally redispersing the lyophilised mPEG-coated MOF nanoparticles, such as in a liquid (e.g. aqueous medium), optionally using sonication.

Preferably, an active ingredient is encapsulated within the one or more mPEG-coated MOF nanoparticles, preferably prior to the step of mixing a suspension of the one or more uncoated MOF nanoparticles with a solution of mPEG-PO₃ and allowing mPEG-PO₃ to coordinate to one or more metal ions of the one or more uncoated MOF nanoparticles to provide a suspension of the one or more mPEG-coated MOF nanoparticles.

Preferably the active ingredient is encapsulated within the one or more mPEG-coated MOF nanoparticles by the step of mixing an active ingredient with a suspension of the one or more uncoated MOF nanoparticles. Typically, the active ingredient may be selected from the group consisting of: pharmaceutically active ingredient, pesticides, insecticides, and dyes.

Preferably, the suspension of the one or more mPEG-coated MOF nanoparticles has a solids content of from about 1 % by weight to about 25% by weight, preferably from about 2% by weight to about 10% by weight, of the total weight of the suspension prior to the lyophilisation step. Preferably, the suspension is an aqueous suspension.

The method according to the seventh aspect of the invention is particularly advantageous as the method may produce a stable suspension of one or more mPEG-coated MOF nanoparticles, which may have improved stability during storage.

In an eighth aspect, the present invention provides a composition produced according to any of the methods disclosed herein, in particular the methods of the fourth, fifth, sixth or seventh aspects of the invention. The composition may be a dry powder pharmaceutical composition comprising one or more lyophilised polymer-coated metal-organic framework (MOF) nanoparticles. Alternatively, the composition may be a liquid suspension of one or more active-containing polymer-coated MOF nanoparticles. Optionally, the composition may be redispersed lyophilised polymer-coated MOF nanoparticles in an aqueous medium.

The compositions according to the eighth aspect of the invention are particularly advantageous because they may deliver improved MOF stability, facilitate lyophilisation and/or facilitate redispersion in a liquid medium. Additionally, the compositions may have improved stability in storage prior to use.

In a ninth aspect, the present invention provides a method of producing a composition comprising one or more polymer-coated active ingredient-containing amorphous metal-organic framework (MOF) nanoparticles. Preferably each MOF nanoparticle is amorphous. Amorphous MOF nanoparticles may be partially and/or substantially fully amorphous. Each nanoparticle comprising a MOF comprising a plurality of metal ions (inorganic nodes) and a plurality of organic ligands.

Preferably, the plurality of metal ions may consist essentially of, or consist of, ions of a metal selected from the group consisting of: zirconium, iron, zinc, hafnium, magnesium or potassium; more preferably the plurality of metal ions may consist essentially of, or consist of, ions of a metal selected from the group consisting of: zirconium and iron; most preferably the plurality of metal ions may consist essentially, or consist of, ions of zirconium.

Preferably, at least one active ingredient may be selected from the group consisting of: pharmaceutically active ingredients, pesticides, insecticides, and dyes.

The particle size of the one or more polymer-coated active ingredient-containing amorphous MOF nanoparticles according to the ninth aspect may be from about 25 nm to about 450 nm, preferably from about 100 nm to about 250 nm, most preferably from about 100 to about 200 nm.

The polymer is attached to one or more metal ions by metal coordination, preferably phosphate-metal coordination. Preferably the polymer includes a phosphate group, more preferably the polymer is phosphate-terminated, preferably wherein the polymer has a single terminal phosphate group. Typically, the polymer comprises a base polymer which has been phosphate functionalised.

Preferably, the polymer (or base polymer) may be selected from the group consisting of polyethylene glycol, hyaluronic acid, heparin, chitosan, polyvinyl alcohol, polyglutamic acid, and derivatives and copolymers thereof.

In embodiments, when the polymer comprises polyethylene glycol or a derivative thereof, preferably the polyethylene glycol is linear polyethylene glycol or a derivative thereof. Preferably the polyethylene glycol is predominantly linear polyethylene glycol or a derivative thereof, more preferably the polymer may consist essentially of, or consists of, linear polyethylene glycol or a derivative thereof. Preferably, the polymer is not a branched polyethylene glycol and/or is substantially free from branched polyethylene glycol.

Preferably, the polyethylene glycol or derivative thereof comprises from about 40 to about 250 repeating units.

Preferably the polymer comprises, or consists essentially of, a phosphate-terminated linear alkoxy polyethylene glycol (PEG), wherein the alkoxy group is preferably a C₁ to C₅ alkoxy, more preferably a C₁ to C₃ alkoxy. Even more preferably, the polymer comprises, or consists essentially of, a phosphate-terminated linear methoxy polyethylene glycol (mPEG).

Preferably, the polymer is a phosphate-terminated mPEG according to the structure A.

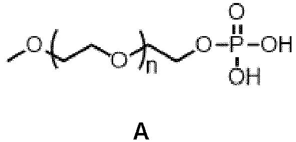

Preferably n is from about 40 to about 250.

Typically, the mPEG (such as the mPEG of structure A) may have a molecular weight of from about 2000 g/mol to about 10000 g/mol (e.g. mPEG2000 to mPEG10000), preferably from about 3000 g/mol to about 9000 g/mol (e.g. mPEG3000 to mPEG9000), from about 4000 g/mol to about 8000 g/mol (e.g. mPEG4000 to mPEG8000), from about 5000 g/mol to about 7000 g/mol (e.g. mPEG5000 to mPEG7000), from about 5000 g/mol to about 6000 g/mol (e.g. mPEG5000 to mPEG6000). Preferably, the mPEG has a molecular weight of about 5000 g/mol (e.g. mPEG5000).

Preferably, the polymer is attached to metal ions located at an external surface of each MOF nanoparticle. Preferably, an external surface of each MOF nanoparticle is substantially coated in the polymer.

The method of the ninth aspect comprises the steps of: i) mixing a plurality of metal ions (inorganic nodes) and a plurality of organic ligands to form a suspension of one or more uncoated MOF nanoparticles; ii) encapsulating an active ingredient within one or more of the uncoated MOF nanoparticles to provide a suspension of one or more active ingredient-containing, uncoated MOF nanoparticles; iii) amorphising the one or more active ingredient-containing, uncoated MOF nanoparticles to form one or more active ingredient-containing, uncoated amorphous (or partially amorphous) MOF nanoparticles; iv) subsequently mixing a suspension of the one or more active ingredient-containing, uncoated amorphous MOF nanoparticles with a phosphate-functionalised polymer and allowing the phosphate-functionalised polymer to coordinate to one or more metal ions of the one or more active ingredient-containing, uncoated MOF nanoparticles to provide a suspension of one or more active ingredient-containing polymer-coated amorphous MOF nanoparticles; v) optionally drying the one or more polymer-coated active ingredient-containing amorphous MOF nanoparticles by lyophilisation; and vi) optionally, subsequently redispersing the lyophilised polymer-coated active ingredient-containing amorphous MOF nanoparticles in an aqueous medium.

Preferably, the amorphising step iii) is carried out by ball-milling, heat treating or irradiating the MOF nanoparticles.

Preferably, the aqueous redispersion medium is selected from the group consisting of: water, bovine serum albumin, phosphate-buffered saline, plasma, and Dulbecco's Modified Eagle Medium (DMEM). Preferably, the aqueous redispersion medium is water.

Preferably, the suspension of the one or more polymer-coated amorphous MOF nanoparticles has a solids content of from about 1% by weight to about 25% by weight, preferably from about 2% by weight to about 10% by weight, of the total weight of the suspension prior to lyophilisation. Preferably, the suspension is an aqueous suspension.

Typically, the coordination step iv) is performed for a period of time (the coating time) sufficient for the metal sites on the external surface of the MOF nanoparticles to be substantially saturated, preferably with polymer with limited or substantially no reduction of the amorphous MOF nanoparticles' porosity. Typically, step iv) lasts less than about three hours, preferably less than about two hours, preferably from about 1 minute to about 180 minutes, preferably from about 30 minutes to about 150 minutes, preferably from about 5 minutes to about 60 minutes.

Preferably, the polymer coating makes up less than about 40 % by weight of the polymer-coated nanoparticle MOF, preferably less than about 30 % by weight, more preferably from about 10 % by weight to about 40 % by weight, preferably from about 20 % by weight to about 35 % by weight of the polymer-coated nanoparticle amorphous MOF.

The method according to the ninth aspect of the invention is particularly advantageous as the method produces a stable suspension of one or more polymer-coated active ingredient-containing amorphous MOF nanoparticles, which may have improved stability during storage.

Additionally, by utilising amorphization active ingredient release from the MOF nanoparticles may be further controlled. Without being bound by theory, it is believed that the combination of polymer coating and amorphization may allow improved control of active ingredient release from the one or more polymer-coated active ingredient-containing amorphous MOF nanoparticles and, in particular, compared to by each technique taken alone.

Moreover, if the one or more polymer-coated active ingredient-containing amorphous MOF nanoparticles are prepared by lyophilisation the composition may be stable during storage and subsequently, on redispersion, provide a suspension of the one or more polymer-coated active ingredient-containing amorphous MOF nanoparticles with reduced aggregation.

In a tenth aspect, the present invention provides the use of a phosphate-functionalised polymer to coat one or more MOF nanoparticles; wherein an active ingredient is located within the one or more MOF nanoparticles being coated.

Preferably, the polymer may be selected from the group consisting of polyethylene glycol, hyaluronic acid, heparin, chitosan, polyvinyl alcohol, polyglutamic acid, and derivatives and copolymers thereof. More preferably, the polymer comprises polyethylene glycol or a derivative thereof.

In embodiments, preferably the polymer is phosphate-terminated, preferably wherein the polymer has a single terminal phosphate group.

In embodiments, when the polymer comprises polyethylene glycol or a derivative thereof, preferably the polyethylene glycol is linear polyethylene glycol or a derivative thereof. Preferably the polyethylene glycol is predominantly linear polyethylene glycol or a derivative thereof, more preferably the polymer may consist essentially of, or consists of, linear polyethylene glycol or a derivative thereof. Preferably, the polymer is not a branched polyethylene glycol and/or is substantially free from branched polyethylene glycol.

Preferably, the polyethylene glycol or derivative thereof comprises from about 40 to about 250 repeating units.

Preferably the polymer comprises, or consists essentially of, a phosphate-terminated linear alkoxy polyethylene glycol (PEG), wherein the alkoxy group is preferably a C₁ to C₅ alkoxy, more preferably a C₁ to C₃ alkoxy. Even more preferably, the polymer comprises, or consists essentially of, a phosphate-terminated linear methoxy polyethylene glycol (mPEG).

Most preferably, the polymer is a phosphate-terminated mPEG according to structure A.

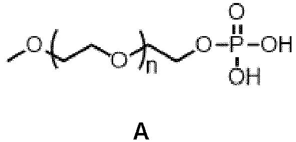

Preferably n is from about 40 to about 250.

Typically, the mPEG may have a molecular weight of from about 2000 g/mol to about 10000 g/mol (mPEG2000 to mPEG 10000), preferably from about 3000 g/mol to about 9000 g/mol (mPEG3000 to mPEG9000), from about 4000 g/mol to about 8000 g/mol (mPEG4000 to mPEG8000), from about 5000 g/mol to about 7000 g/mol (mPEG5000 to mPEG7000), from about 5000 g/mol to about 6000 g/mol (mPEG5000 to mPEG6000). Preferably, the mPEG has a molecular weight of about 5000 g/mol (mPEG5000). Typically, the polymer is attached to the one or more metal ions by phosphate-metal coordination.

The use according to the tenth aspect of the invention is particularly advantageous as it may improve MOF nanoparticle stability, facilitate lyophilisation and/or facilitate redispersion in a liquid medium.

In an eleventh aspect, the present invention provides the use of a polymer to improve redispersion of a plurality of lyophilised MOF nanoparticles, wherein the polymer is coated onto the MOF nanoparticles prior to lyophilisation.

Preferably, the polymer may be selected from the group consisting of polyethylene glycol, hyaluronic acid, heparin, chitosan, polyvinyl alcohol, polyglutamic acid, and derivatives and copolymers thereof. More preferably, the polymer comprises polyethylene glycol or a derivative thereof.

In embodiments when the polymer comprises polyethylene glycol or a derivative thereof, preferably the polyethylene glycol is linear polyethylene glycol or a derivative thereof, preferably the polyethylene glycol is predominantly linear polyethylene glycol or a derivative thereof, more preferably the polymer may consist essentially of, or consist of, linear polyethylene glycol or a derivative thereof. Preferably, the polymer is not a branched polyethylene glycol, or a derivative thereof.

Preferably, the linear polyethylene glycol comprises from about 40 to about 250 repeating units.

Preferably the polymer comprises, or consists essentially of, a phosphate-terminated linear alkoxy polyethylene glycol (PEG), wherein the alkoxy group is preferably a C₁ to C₅ alkoxy, more preferably a C₁ to C₃ alkoxy. Even more preferably, the polymer comprises, or consists essentially of, a phosphate-terminated linear methoxy polyethylene glycol (mPEG).

Most preferably, the polymer is a phosphate-terminated mPEG according to structure A.

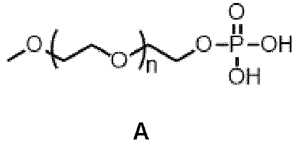

Preferably n is from about 40 to about 250.

Typically, the mPEG may have a molecular weight of from about 2000 g/mol to about 10000 g/mol (mPEG2000 to mPEG 10000), preferably from about 3000 g/mol to about 9000 g/mol (mPEG3000 to mPEG9000), from about 4000 g/mol to about 8000 g/mol (mPEG4000 to mPEG8000), from about 5000 g/mol to about 7000 g/mol (mPEG5000 to mPEG7000), from about 5000 g/mol to about 6000 g/mol (mPEG5000 to mPEG6000). Preferably, the mPEG has a molecular weight of about 5000 g/mol (mPEG5000). Typically, polymer is attached to the one or more metal ions by metal coordination, preferably phosphate-metal coordination.

The use according to the eleventh aspect of the invention is particularly advantageous as redispersion of a plurality of MOF nanoparticles may be improved.

In a twelfth aspect, the present invention provides a method of treating a disease or condition by administering a composition according to the first, second, third and eighth aspects of the invention to a patient.

### Brief Description of the Drawings

The invention will now be described with reference to the following figures which are intended to be non-limiting.
**Figure 1** Schematic illustration of the synthesis of PCN-222 and PCN-222@PEG-PO₃.
**Figure 2** Simulated PXRD pattern of PCN-222 and experimental PXRD patterns of PCN-222, PCN-222@PEG-PO₃ and mPEG-PO₃.
**Figure 3** ³¹P SSNMR spectra of PCN-222@PEG-PO₃ and mPEG-PO₃.
**Figure 4** XPS survey spectra of PCN-222@PEG-PO₃ and PCN-222.
**Figure 5** High-resolution Zr 3d spectra of PCN-222 and PCN-222@PEG-PO₃.
**Figure 6** Intensity-average diameter of the suspension of PCN-222 (green line), PCN-222@PEG-PO₃ (blue line) and the re-dispersed solution of PCN-222@PEG-PO₃ (pink line) in water (n = 3).
**Figure 7** Zeta potential of water suspensions of PCN-222 and PCN-222@PEG-PO₃.
**Figure 8** TEM images of drop cast PCN-222.
**Figure 9** TEM images of drop cast PCN-222@PEG-PO₃.
**Figure 10** SEM image of lyophilized PCN-222@PEG-PO₃, inset is the optical images of lyophilized PCN-222@PEG-PO₃ (left) and its re-dispersed suspension (right). Scale bar, 500 nm.
**Figure 11** Proposed schematic of PCN-222@PEG-PO₃ formation.
**Figure 12** Simulated and experimental PXRD patterns. Characterization recorded at different intervals of time (2 h, 4 h, 12 h and 16 h).
**Figure 13** N₂ isotherms at 77K. Characterization recorded at different intervals of time (2 h, 4 h, 12 h and 16 h).
**Figure 14** PSD obtained with the NLDFT method. Characterization recorded at different intervals of time (2 h, 4 h, 12 h and 16 h).
**Figure 15** Simulated and experimental PXRD patterns of PCN-222 (Hf).
**Figure 16** HAADF-STEM image of PCN-222 (Hf). Scale bar, 500 nm.
**Figure 17** Simulated PXRD patterns of UiO-66, MOF-808, NU-901 and PCN-128 and experimental PXRD patterns of UiO-66 and UiO-66@PEG-PO₃, MOF-808 and MOF-808@PEG-PO₃, NU-901 and NU-901@PEG-PO₃, and PCN-128 and PCN-128@PEG-PO₃.
**Figure 18** SEM image of lyophilised UiO-66@PEG-PO₃.
**Figure 19** SEM image of lyophilised MOF-808@PEG-PO₃.
**Figure 20** SEM image of lyophilised NU-901@PEG-PO₃.
**Figure 21** SEM image of lyophilised PCN-128@PEG-PO₃.
**Figure 22** Intensity-average diameter of the suspension of parent nanoMOFs (green line), MOF@PEG-PO₃ (blue line) and the re-dispersed suspension of MOF@PEG-PO₃ (pink line) in water (n = 3). MOF = UiO-66, MOF-808, NU-901 and PCN-128. Scale bar, 500 nm.
**Figure 23** **¹****H** NMR spectrum of mPEG-PO₃.
**Figure 24** ³¹P NMR spectrum of mPEG-PO₃.
**Figure 25** GPC trace of mPEG-PO₃ (5k).
**Figure 26** SEM images of PCN-222 in different sizes, dependent on TFA addition.
**Figure 27** TEM and HAADF-STEM (inset) images of PCN-222. Scale bar, 500 nm (left), 200 nm (right) (inset, 15 nm).
**Figure 28** TEM and HAADF-STEM (inset) images of PCN-222@PEG-PO₃.Scale bar, 500 nm (left), 200 nm (right) (inset, 15 nm).
**Figure 29** SEM images of **a** UiO-66, **b** MOF-808, c NU-901 and **d** PCN-128. **b** inset, 300 nm.
**Figure 30** Optical images of **a** air-dried and **b** lyophilised PCN-222@PEG-PO₃ with the same mass (60 mg).
**Figure 31** SEM images of air-dried PCN-222@PEG-PO₃ at different magnifications.
**Figure 32** SEM images of lyophilized PCN-222@PEG-PO₃ at different magnifications.
**Figure 33** FT-IR spectra: **a** full spectra and **b** the enlarged spectra.
**Figure 34** TGA profiles of PCN-222@PEG-PO₃: **a** PEGylation 2 h, **b** PEGylation 4 h, **c** PEGylation 12 h and **d** PEGylation 16 h.
**Figure 35** P 2p XPS spectra of PCN-222@PEG-PO₃.
**Figure 36** XPS spectra of PCN-222 (Hf) and PCN-222(Hf)@PEG-PO₃. **a** XPS survey spectrum and **b** high-resolution of P 2p spectrum of PCN-222(HD@PEG-PO₃.
**Figure 37** N₂ sorption isotherms and PSD of PCN-222 and PEGylation after 2 h. **a** and **b** before, **c** and **d** after subtracting the amount of mPEG-PO₃ included.
**Figure 38** N₂ sorption isotherms and PSD of PEGylation after 4, 12 and 16 h. **a** and **b** before, **c** and **d** after subtracting the amount of mPEG-PO₃ included.
**Figure 39** N₂ sorption isotherms and PSD of PCN-222 and PEGylation after 16 h. **a** and **b** before, c and d after subtracting the amount of mPEG-PO₃ included.
**Figure 40** N₂ sorption isotherms of PCN-222 (Hf).
**Figure 41** N₂ isotherms and PSD of MOF@PEG-PO₃. **a** UiO-66@PEG-PO₃, **b** MOF-808@PEG-PO₃@PEG-PO₃, **c** NU-901@PEG-PO₃ and **d** MOF-128@PEG-PO₃.
**Figure 42** Long-term dispersity of bare MOFs (green line) and MOF@PEG-PO₃ suspended in H₂O and PBS, respectively. n=3.
**Figure 43** Doxorubicin (DOX) controlled release profile from PCN-222 and PCN-222@PEG-PO₃.
**Figure 44** UV-Vis spectra of DOX; before and after MOF encapsulation, PEGylation
**Figure 45** Cumulative release profiles of DOX from DOX@MOF and DOX@MOF@PEG-PO₃ in PBS (pH = 7.4)

### Detailed Description

The present invention relates to metal-organic framework (MOF) nanoparticles and, in particular, coated MOF nanoparticles, methods of manufacturing said coated MOF nanoparticles, and uses of said coated MOF nanoparticles.

Metal-organic frameworks (MOFs) are crystalline materials consisting of coordination bonds between metal ions, e.g. metal cations, and multidentate organic ligands. The MOF structure is characterised by an open framework that may be porous.

MOF nanoparticles comprise metal-organic frameworks and may display 3D architectures. MOF nanoparticles disclosed herein may be generally rod-shaped, spherical, or cuboid. Preferably, the MOF nanoparticles disclosed herein are generally rod-shaped. Polymer-coated MOF nanoparticles of the present invention may each comprise a MOF comprising a plurality of metal ions (inorganic nodes) and a plurality of organic ligands, wherein polymer is attached to one or more metal ions of each nanoparticle.

Preferred MOF nanoparticles according to the present invention may be PCN-222, UiO-66, MOF-808, NU-901 and PCN-128, more preferably PCN-222. Nomenclature for coated MOF nanoparticles is MOF@ polymer. For example, PCN-222 wherein the polymer coating is mPEG attached via phosphate coordination is denoted as PCN-222@mPEG-PO₃.

The particle size of the one or more polymer-coated MOF nanoparticles is from about 25 nm to about 450 nm. For the avoidance of doubt, unless otherwise stated, the particle size disclosed herein relates to the coated MOF nanoparticles. Preferably the particle size of the one or more polymer-coated MOF nanoparticles is from about 100 nm to about 250 nm, more preferably from about 100 nm to about 200 nm.

The particle size of the one or more polymer-coated MOF nanoparticles may be measured according to the dynamic light scattering (DLS) method disclosed herein. The particle size refers to the hydrodynamic diameter, which can be defined as the radius of a hypothetical sphere that diffuses at the same rate as the particle under investigation.

Preferably, the uncoated MOF nanoparticles are substantially monodisperse. The uncoated MOF nanoparticles may, for instance, have a polydispersity index of less than about 0.250 nm, less than about 0.120 nm, preferably from about 0.100 nm to about 0.250 nm.

Typically, a MOF nanoparticle contains only a single species of the metal ion. Accordingly, the plurality of metal ions (inorganic nodes) may consist essentially of (or consist of) ions of a metal selected from the group consisting of zirconium, iron, zinc, hafnium, magnesium and potassium. The plurality of metal ions (inorganic nodes) may consist essentially of (or consist of) an ion of a metal selected from the group consisting of zirconium and iron. Preferably, the plurality of metal ions consists essentially of (or consists of) ions of zirconium. Typically, the metal ion selected will be biocompatible/pharmaceutically acceptable.

The organic ligands in the MOFs according to the present invention may be multidentate. A multidentate organic ligand is an organic ligand capable of donating two or more pairs of electrons in a complexation reaction to form two or more coordinate bonds. The organic ligands may comprise two or more oxygen and/or nitrogen atoms suitable for donating a pair of electrons to form the two or more coordinate bonds. Preferably, the oxygen atoms may be present as carboxylate or nitro groups. Preferably, the nitrogen atoms may be present as amine groups. Typically, the plurality of organic ligands may be an aromatic carboxylate, an aromatic amine and/or an aromatic nitro. Preferably, the plurality of organic ligands may be selected from the group consisting of: tetrakis (4-carboxyphenyl)porphyrin (TCPP), 1,4-benzenedicarboxylic acid (BDC), 1,3,5-benzenetricarboxylic acid (H₃BTC), 1,3,5,8- (p-benzoate)pyrene linkers (H₄TBAPy), 4',4''',4''''',4'''''''-(ethene-1,1,2,2- tetrayl)tetrakis(([1,1'-biphenyl]-3-carboxylic acid)) (H₄ETTC). Typically, the organic ligand selected will be biocompatible/pharmaceutically acceptable.

Polymer may be attached to one or more metal ions of each nanoparticle by phosphate-metal coordination. Advantageously, this provides a relatively strong bond without necessitating complex chemistry. Polymer for the purposes of the invention may have 40 or more repeat units and may include homopolymers and copolymers, including alternating, periodic, random, block and graft copolymers. Preferably the polymers are substantially linear. Branch polymers are typically not preferred. Typically, the polymer selected will be biocompatible/pharmaceutically acceptable.

Generally, oligomers are not included. For the purposes of the invention, an oligomer is understood to be a molecule comprising a number of repeat units such that the molecule's properties vary significantly with the removal of one or a few of the units. An oligomer may comprise 30 or less repeat units; for instance, less than 10 repeat units. In particular, oligonucleotides and cyclodextrins may be excluded.

Preferably, the polymer may be selected from the group consisting of polyethylene glycol, hyaluronic acid, heparin, chitosan, polyvinyl alcohol, polyglutamic acid, and derivatives and copolymers thereof. Typically, the polymer comprises a phosphate group, preferably a terminal phosphate group. Preferably, the polymer comprises substantially no branching.

Preferably the base polymer is hydrophilic.

The polymer may have a molecular weight (M_{w}) of from about 2000 g/mol to about 10000 g/mol, preferably from about 3000 g/mol to about 9000 g/mol, from about 4000 g/mol to about 8000 g/mol, from about 5000 g/mol to about 7000 g/mol, from about 5000 g/mol to about 6000 g/mol. Molecular weight may be determined using gel permeation chromatography as described herein.

More preferably, the polymer comprises polyethylene glycol or a derivative thereof, preferably a phosphate-terminated alkoxy polyethylene glycol (alkoxy PEG), wherein the alkoxy group is preferably a C₁ to C₅ alkoxy, preferably a C₁ to C₃ alkoxy. Even more preferably, the polymer comprises a phosphate-terminated methoxy polyethylene glycol (mPEG).

Most preferably, the polymer is a phosphate-terminated mPEG according to structure A. Preferably n is from about 40 to about 250.

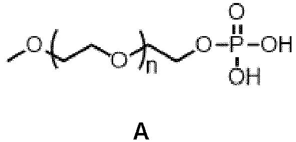

Without wishing to be bound by theory, it is believed that phosphate-terminated polymer displaces any active ingredients adsorbed to the surface of the MOF nanoparticle(s). This is particularly advantageous as they may clear superfluous active ingredient from the surface of the MOF nanoparticle(s), while retaining the active ingredient encapsulated within the MOF nanoparticle(s), and may aid in controlling active ingredient release from the MOF nanoparticle(s). Accordingly, without being bound by theory, it may be particularly advantageous to perform the active ingredient encapsulation step prior to the polymer coating step.

An active ingredient is an agent that produces an intended effect under conditions of the intended use of the MOF nanoparticle, typically in an environment external to the MOF nanoparticle. Active ingredients according to the present invention may be pharmaceutically active ingredients, pesticides, insecticides, perfumes, and dyes.

A pharmaceutically active ingredient is an ingredient that has a therapeutic or prophylactic effect on diseases or conditions of the human or animal body.

Non-limiting examples of pharmaceutically active ingredients suitable for use in the present invention include antibiotics, analgesics, alpha-blockers, anti-allergy, anti-asthma, (allergic rhinitis, chronic urticaria), anti-inflammatory, antacids, anthelmintics, anti-arrhythmic agents, anti-arthritis, anti-bacterials, anti-anxiety, anti-coagulants, anti-depressants, antidiabetics, anti-diarrheals, anti-diuretics, anti-epileptics, anti-fungal, anti-gout, anti-hypertensive, anti-incontinence, anti-insomnia, anti-malarials, anti-migraine, anti-muscarinic, anti-neoplastic and immunosuppressants, anti-protozoal, anti-rheumatics, anti-rhinitis, anti-spasmatic. anti-thyroid, antivirals, anxiolytics, sedatives, hypnotics and neuroleptics, betablockers, anti-benign hyperplasia (BHP), cardiac inotropic, corticosteroids, cough suppressants, cytotoxics, decongestants, diuretics, enzymes, anti-parkinsonian, gastrointestinal, histamine receptor antagonists, hormone replacement therapy, lipid regulating agents, local anesthetics, neuromuscular agents, nitrates and anti-anginal agents, anti-pain, anti-nausea, nutritional agents, opioid analgesics, vaccines, proteins, peptides and recombinant drugs, prevention of chemotherapy-induced and post-operative nausea and vomiting proton pump inhibitors, sex hormones and contraceptives, spermicides, veterinary medicines, treatments for diabetic gastric stasis, infertility, endometriosis, menstrual disorders, motion sickness, movement disorders, schizophrenia, seizure/panic disorder, sexual dysfunction (male and female), stimulants voiding dysfunctions, and the like or combinations thereof. The use of cytotoxic pharmaceutically active ingredients useful in the treatment of cancer is particularly preferred.

The pharmaceutical composition may be provided as a solid, a suspension, preferably a dry powder, or an aqueous suspension. Suspensions herein include colloidal suspensions. Preferably the suspension(s) is(are) a colloidal suspension(s). This may be confirmed by using a red laser to observe the Tyndall effect.

The pharmaceutical composition may further comprise one or more pharmaceutically acceptable excipients. Preferably, the excipient is selected from the group consisting of: solvents, co-solvents, buffers, stabilisers, antioxidants, preservatives, chelating agents, emulsifiers, flavourings, lubricants, suspending agents, tonicity adjusting agents, surfactants, solubilisers, suspending aids, dispersion agents, humectants, thickeners, colouring agent, wetting agent, anti-foaming agent, viscosity modifier, sweeteners and combinations thereof.

The dry powder pharmaceutical compositions of the invention may comprise from about 50 wt% to about 100 wt%, from about 60 wt% to about 90 wt%, from about 70 wt% to about 80 wt% as a percentage of the entire composition of the pharmaceutically active ingredient-containing lyophilised polymer-coated MOF nanoparticles.

Preferably, the pharmaceutically active ingredient-containing lyophilised polymer-coated MOF nanoparticles releases less than about 30 wt% of the active ingredient over the first six hours in aqueous solution, preferably less than about 25 wt% of the active ingredient, more preferably less than about 20 wt%. Typically, the aqueous solution is dulbelocco's PBS, pH 7.4, at 25 °C.

Methods of administration of a pharmaceutical composition according to the present invention include oral, parenteral (for example, intravenous, subcutaneous, intramuscular or intraarticular), cutaneous (for example, topical) or inhalation administration, intravenous being particularly preferred. The pharmaceutical composition may be a liquid composition.

Lyophilisation (also known as freeze drying or cryodesiccation) is a drying process carried out at low temperature. Lyophilisation generally involves reducing temperature and pressure to below the substance's triple point and removing the frozen solvent (e.g. water ice) by sublimation. For aqueous compositions, such as those disclosed herein, lyophilisation may be carried out at temperatures of from about -50 °C to -80 °C, preferably about -70 °C, and pressures of from about 1000 Pa (0.01 bar) to about 100 Pa (0.001 bar), preferably about 800 Pa (0.008 bar).

Preferably, lyophilisation is performed on a concentrated aqueous slurry of the polymer-coated MOF nanoparticles, typically wherein the aqueous slurry is lyophilised in a plurality of vials having a volume of from about 5 ml to about 25 ml.

Redispersion is the process of dispersing a previously dried solid material (for instance the lyophilised composition(s) of the invention) into suspension for a second or further time. Preferably the redispersed suspension is a colloidal suspension. Typically, the colloidal suspension is stable for at least one week.

When the polymer coated nanoparticle MOFs contain an encapsulated active ingredient, preferably they are washed between the condensing step and lyophilisation to remove excess active ingredient which has not been encapsulated. Typically, they are washed using water.

### Examples

The invention will now be demonstrated by reference to the following non-limiting examples.

Unless otherwise mentioned, room temperature and pressure are 20 °C (293.15 K, 68 °F) and 1 atm (14.696 psi, 101.325 kPa), respectively.

### Experimental Methods

When referred to herein the following methods and procedures were employed.

### Powder X-ray Diffraction (PXRD)

Powder X-ray diffraction (PXRD) data were collected on a Bruker D8 DAVINCI diffractometer at 298 K (24.85 °C) using Cu K*α* radiation. The calculated PXRD patterns were produced using the Mercury program and single crystal reflection data.

### Thermogravimetric Analysis (TGA)

Measurements were carried out using a TA Instruments Q500 Thermogravimetric Analyzer. Measurements were collected from room temperature to 800 °C with a heating rate of 5 °C/min under nitrogen.

### Gas Uptake

N₂ adsorption isotherm measurements were performed on a Micromeritics 3Flex analyzer at 77 K (-196.15 °C). Samples were degassed under vacuum at 120 °C for 20 hours using the internal turbopump. The BET areas were estimated by applying the Brunauer-Emmett-Teller (BET) equation.

### Dynamic Light Scattering (DLS)

Measurements were recorded in a water suspension on a Zetasizer Nano ZS, (Malvern Instrument Ltd., U.K.) equipped with a He-Ne laser operating at 633 nm at 25 °C and analysed using Zetasizer Software.

### Inductively coupled plasma-optical emission spectroscopy (ICP-OES)

ICP-OES was performed using a Perkin Elmer ICP-OES Optima 2100DV. Samples were dispersed in 2 mL of nitric acid and 6 mL of hydrochloric acid, and left to stand at room temperature in the fume cupboard for at least 1 h until all reactions have ceased. After that, samples were heated at 90 °C for 10 h to fully digest the sample. The mixture was diluted 750 times before the measurement.

### X-ray photoelectron spectroscopy (XPS)

XPS analysis was carried out using a Thermo Fisher Scientific ESCALAB 250Xi XPS System. A monochromatic Al-Kα source (1486.74 eV) and a charge neutralizer were used for all samples. Survey scans were acquired using pass energy of 100 eV, 1 scan and dwell time of 50ms. High-resolution data were acquired using 50 eV pass energy, 50 scans, dwell time of 50ms and standard magnetic lenses.

### Liquid Nuclear Magnetic Resonance Spectroscopy (NMR)

Liquid NMR was carried out using a Bruker 400 MHz Avance III HD Smart Probe Spectrometer.

### Solid-State Nuclear Magnetic Resonance Spectroscopy (NMR)

Solid-state NMR experiments were conducted on a Bruker Avance III 200 MHz spectrometer equipped with a 4.7 T magnet (corresponding to Larmor frequencies: v₀(¹H) = 200 MHz and v₀(³¹P) = 81.0 MHz). A Bruker 1.3 mm double-channel magic-angle spinning (MAS) probe was used with a spinning frequency of 60 kHz. Spectra were acquired using a rotor-synchronized Hahn echo experiment with optimized excitation and refocusing pulse lengths of 1.56 and 3.12 µs, respectively. A recycle delay of 1 s was used for all experiments, and either 4096 (mPEG-PO₃ sample) or 8192 (MOF sample) transients were collected. Spectra were processed with 50 Hz of exponential apodization, zero filling, followed by Fourier transformation. ³¹P chemical shifts were calibrated using 85% H₃PO₄ in H₂O (6ᵢₛₒ(³¹P) = 0 ppm) as an external reference.

### UV-Vis/Fluorescence Spectroscopy

UV-vis and fluorescence spectra were recorded using a Tecan Spark^{®} Multimode Microplate Reader.

### Fourier-transform infrared spectroscopy (FT-IR)

FT-IR was carried out using a Bruker Tensor 27 FTIR with attenuated total reflectance (ATR) method.

### Gel Permeation Chromatography (GPC)

GPC was carried out using a Shimadzu HPLC system consisting of an LC-20AD Pump, SIL-20A autosampler, CTO-20A column oven and CBM-20A control unit.

### Scanning Electron Microscopy (SEM)

The samples for SEM tests were coated with Pt or Au for 40 seconds and imagined using a FEI Nova Nano SEM 450.

### Transmission electron microscopy (TEM)

The samples for TEM test were prepared by dispersing the samples in ethanol using ultrasonication. After that, a small number of suspensions were drop-casted on a copper grid with a carbon support film. TEM micrographs were collected on a Tecnai F20 with an acceleration voltage of 200 kV.

### High-Angle Annular Dark-Field Scanning Transmission Electron Microscopy (HAADF-STEM) imaging

Samples were prepared by wet dispersion from aqueous solution. Scanning transmission electron microscopy (STEM) was carried out in an FEI Tecnai Osiris operated at 200 kV using an OneView CMOS camera. The energy-dispersive X-ray (EDX) chemical maps were acquired using a Super-X detector setup, with a total acquisition solid angle of 0.9 sr. EDX data were analysed using ES Vision.

### Size exclusion chromatography with multi-angle light scattering (SEC-MLAS)

SEC-MLAS was performed on a Shimadzu HPLC system consisting of an LC-20AD Pump, SIL-20A autosampler, CTO-20A column oven and CBM-20A control unit. The column set was made up of 1 x PSS SUPREMA analytical 100Å (8x300 mm) and 2xPSS SUPREMA analytical 3000Å (8 x 300 mm). Dual detection was achieved *via* a Wyatt DAWN HELEOS-II multiangle light scattering (MALS) detector (laser at λ = 658 nm), and Wyatt Optilab rEX differential refractive index (DRI) detector with a 658 nm light source. MilliQ Water containing 0.1 mol/L sodium nitrate and 0.01 mol/L sodium azide was used as the eluent at a flow rate of 1.0 mL/min. The column temperature and the detector temperature were kept at 30 °C. All data analysis was performed using Wyatt Astra V 6.1.1 software. A literature value for the dn/dc of poly(ethylene glycol) in water (0.134 ml/g) (see Hasse et al., Macromolecules 1995, 28 (10), 3540-3552) was used to determine the molecular weight of all samples.

### Molecular Dynamics (MD) Simulations

The structure of PCN-222 is taken from the work of Feng et al (Angew. Chem. Int. Ed. 2012, 51 (41), 10307-10310.). The structure of the mPEG-PO₃ (consisting of 128 monomer units) is created in Material Studio. Geometry optimization of both the polymer and the framework is performed using the Forcite Module in Materials Studio. Charges for the framework and the PEG are calculated using the EQeq protocol (see Wilmer et al., J. Phys. Chem. Lett. 2012, 3 (17), 2506-2511.). All simulations are performed using the LAMMPS (Plimpton, J. Comput. Phys. 1995, 117 (1), 1-19.) molecular dynamics package using the Universal Force Field (UFF) (Rappe et al., J. Am. Chem. Soc. 1992, 114 (25), 10024-10035) parameters to describe the Lennard-Jones, bond, angle, dihedral and improper torsion potentials. The simulation box consists of a 2x2x2 cell of PCN-222 -which is considered large enough to avoid any finite-size effects - and one mPEG-PO₃ chain placed at the external surface of the framework. Periodic boundary conditions are applied in all three dimensions. Initially, energy minimization (equilibration run) of the model is done using the NVE ensemble until a temperature of 298K (24.85 °C) is reached. Next, the production runs are conducted using the canonical ensemble (NVT) for a duration of 1.5 ns. All MD simulations are done using a time step of 1 fs. The temperature is maintained using a Nose-Hoover thermostat (Evans et al., J. Chem. Phys. 1985, 83 (8), 4069-4074) with a damping factor of 0.1 ps. The long-range electrostatic interactions are computed using Ewald summation and the precision is set to 1 x 10⁻⁴. The non-bonded van-der Waals interactions are computed using the Lennard-Jones potential and the short-range electrostatic interactions are computed using the coulombic potential, both with a cut off of 12.5 A. The trajectories are sampled every 0.01 ns and are viewed using VMD (see Humphrey et al., J. Mol. Graph. 1996, 14 (1), 33-38).

### Materials and Synthesis

All reagents unless otherwise stated were obtained from commercial sources and were used without further purification.

### Synthesis of mPEG5K-phosphate (mPEG-PO₃)

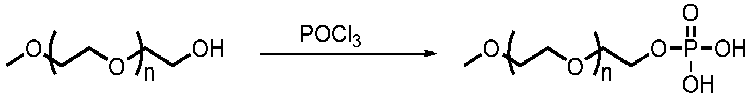

Poly(ethylene glycol) methyl ether (Mₙ = 5000, 37.0 mmol) was dissolved in 150 mL of dry tetrahydrofuran (THF), then POCl3 (44.4 mmol, 1.2 equiv) was added dropwise with stirring. The resulting solution was heated to 60 °C for 10 h under N₂ flow to remove HCI that was formed during the reaction. After cooling to room temperature, distilled water (40 ml) was added, then the mixture was reheated to 60 °C for an additional 3 h and cooling again to room temperature. THF was removed under a vacuum, and the raw product was purified by dialysis using dialysis tubing with a 3,500 molecular weight cutoff, followed by removing the residual water to give mPEG-PO₃ as a white solid (154.8 g, 31.45 mmol, 85%). ¹H NMR (D₂O, 400 MHz), δ: 3.63 (s, 448H, -CH₂OCH₂-), 3.31 (s, 3H, -OCH₃) ³¹P NMR δ: (D₂O, 162 MHz) δ: 0.22.

### Synthesis of Zr₆ cluster

The Zr₆ cluster was synthesized according to the reported procedure (Noh et al., Chem. Mater. 2018, 30 (7), 2193-2197). 15 ml of a 80 % solution of Zr(OBu)₄ (5 mmol) in n-Butanol was added into a solution of 100 g (818.8 mmol) benzoic acid in 300 ml of n-propanol, resulting in a clear mixture solution. The mixture is further refluxed for overnight and white solid precipitation appears. After filtration, 10.2 g (yield: 63 %) solid was separated, washed by anhydrous n-propanol five times and dried under vacuum.

### Size-Controlled Synthesis of PCN-222 Nanoparticles

Tetrakis (4-carboxyphenyl)porphyrin (TCPP) (22.5 mg, 28.5 µmol), Zr₆ cluster (38 mg, 14.2 µmol), 8 mL DMF and **X µL of TFA** were ultrasonically dissolved in a 20 mL threaded vial. The resultant mixture was placed in the 120 °C block heater for 5 hours. After cooling down to room temperature, the obtained purple sample was collected by high-speed centrifugation, followed by washing with fresh dimethylformamide (DMF) 3 times and exchanging with ethanol 3 times, the final product was re-dispersed in ethanol for further use. (X = 80, 130, 180 and 400 µL, the related size = -80, -120, -280 and -620 nm).

To control the size of PCN-222, different contents of trifluoroacetic acid (TFA) may be utilized.

### Synthesis of PCN-222 (Hf)

TCPP (10 mg, 12.6 µmol), HfOCl₂.8H₂O (25 mg, 62.1 µmol), 8 mL DMF and 200 µL TFA were ultrasonically dissolved in a 20 mL threaded vial. The resultant mixture was placed in the 120 °C block heater for 5 hours. After cooling down to room temperature, the obtained purple sample was collected by high-speed centrifugation, followed by washing with fresh DMF 3 times and exchanging with ethanol 3 times, the final product was re-dispersed in ethanol for further use.

### Synthesis of UiO-66 (∼ 430 nm)

1,4-benzenedicarboxylic acid (BDC) (320 mg, 1.93 mmol), ZrCl₄ (466 mg, 2.00 mmol), benzoic acid (2.44 g, 20 mmol) and 36 mL DMF were ultrasonically dissolved in a 100 mL threaded vial, followed by addition of concentrated HCI (0.33 mL). The mixture was heated at 120 °C for 48 h. After cooling to room temperature, the obtained sample was collected by high-speed centrifugation, followed by washing with fresh DMF 3 times and exchanging with ethanol 3 times, the final product was re-dispersed in ethanol for further use.

### Synthesis of MOF-808 (∼ 90 nm)

1,3,5-benzenetricarboxylic acid (70 mg, 0.33 mmol), ZrCl₄ (233 mg, 1.00 mmol), formic acid (5.6 mL) and DMF (10 mL) were ultrasonically dissolved in a 50 mL threaded vial. The mixture was heated at 120 °C for 48 h. After cooling to room temperature, the obtained sample was collected by high-speed centrifugation, followed by washing with fresh DMF 3 times and exchanging with ethanol 3 times, the final product was re-dispersed in ethanol for further use.

### Synthesis of NU-901 (∼ 240 nm)

1,3,6,8-tetrakis(p-benzoic acid)pyrene (H₄TBAPy) (25 mg, 40.4 µmol), ZrOCl₂·8H₂O (121 mg, 375.5 µmol), 4-aminobenzoic acid (400 mg, 2.92 mmol), TFA (200 µL) and DMF (20 mL) were ultrasonically dissolved in a 50 mL threaded vial. The mixture was heated at 140 °C for 50 min, whereupon a yellow suspension formed. After cooling to room temperature, the obtained sample was collected by centrifugation, followed by washing with fresh DMF 3 times and exchanging with ethanol 3 times, the final product was re-dispersed in ethanol for further use.

### Synthesis of PCN-128 (∼ 160 nm)

(4', 4''', 4''''', 4'''''''-(ethene-1,1,2,2-tet-rayl)tetrakis(([1,10-biphenyl]-4-carboxylate) (ETTC) (10 mg, 12.3 µmol), Zr₆ cluster (20 mg, 7.5 µmol), DMF (4 mL) and TFA (100 µL) were ultrasonically dissolved in a 6 mL threaded vial. Afterwards, triethylamine (TEA) (20 µL) was added. The mixture was heated at 120 °C for 24 h. After cooling to room temperature, the obtained sample was collected by high-speed centrifugation, followed by washing with fresh DMF 3 times and exchanging with ethanol 3 times, the final product was re-dispersed in ethanol for further use.

### Size-Controlled Synthesis and Characterization of PCN-222

PCN-222 is an example of a Zr(IV)-based porphyrinic MOF. PCN-222 consists of Zr₆ clusters with eight edges connected to the tetrakis (4-carboxyphenyl)porphyrin (TCPP) linkers, featuring 1D micro- and mesoporous channels with diameters of 1.7 nm and 3.6 nm, respectively.

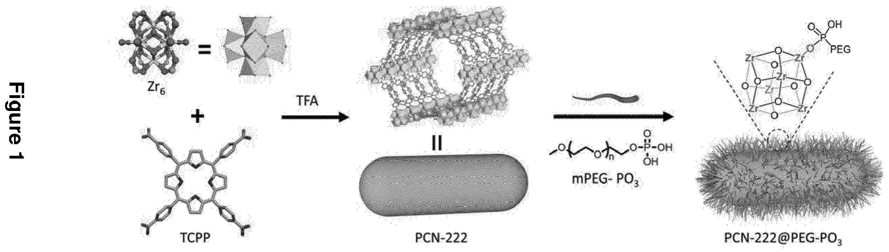

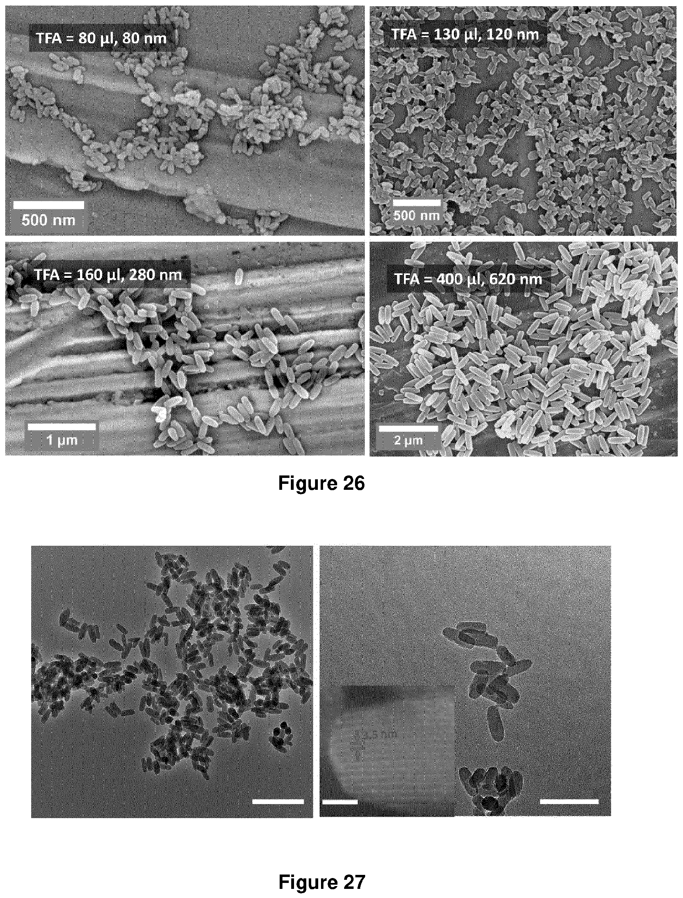

First, the Zr₆ clusters were synthesised as described above. NanoPCN-222 was prepared through a solvothermal reaction between TCPP and Zr₆ clusters using trifluoroacetic acid (TFA) as a modulator as described hereinbefore (Figure 1). PCN-222 size can be modulated by tuning the amount of TFA employed. With higher amounts of TFA, nanoPCN-222 with increased particle size can be obtained (Figure 26). A nanoPCN-222 with a particle size of about 120 nm was chosen.

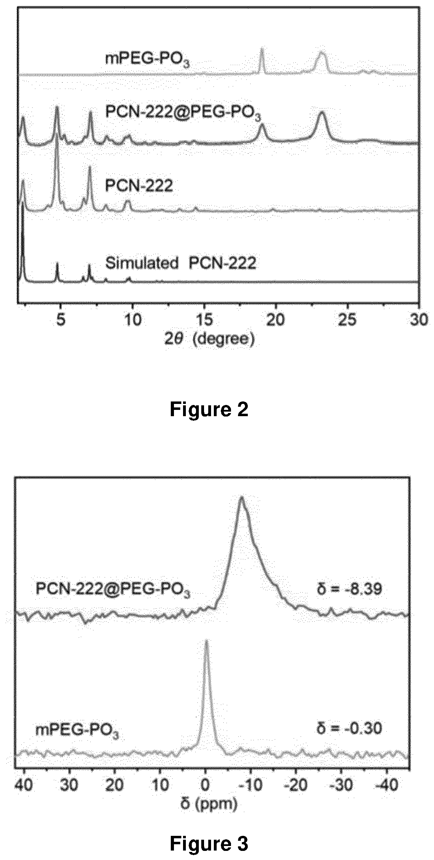

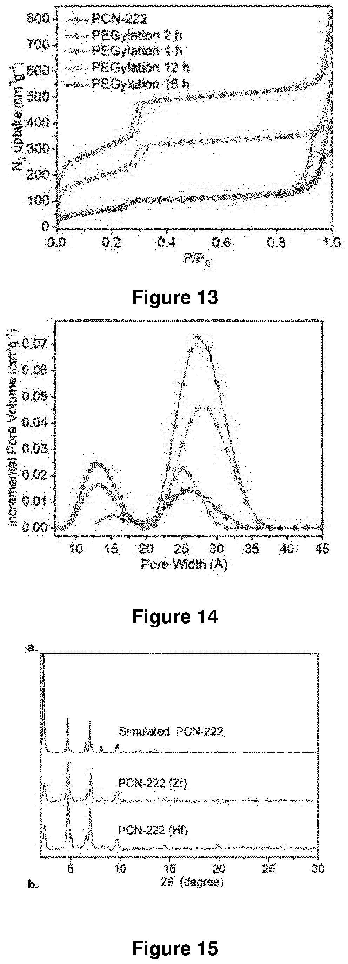

Powder X-ray diffraction (PXRD) showed the presence of broad peaks that match the pattern predicted (simulated) from the single crystal structure, confirming the phase purity of nanoPCN-222 (Figure 2). Figure 13 exhibits a typical Type IV N₂ adsorption isotherm at 77 K for PCN-222.

Transmission electron microscopy (TEM) imaging shows that the PCN-222 nanoparticles have a rod-shaped morphology and size uniformity, whereas the high-angle annular dark-field scanning transmission electron microscopy (HAADF-STEM) imaging confirms the existence of highly-oriented mesopores (Figure 27).

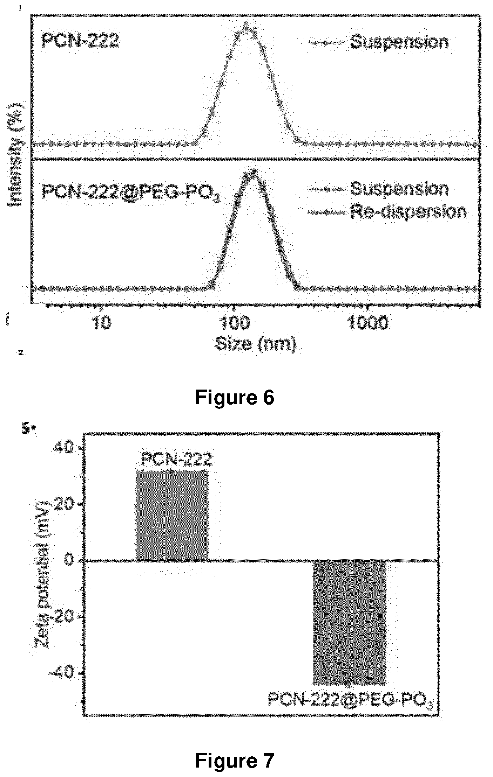

Dynamic light scattering (DLS) of PCN-222 showed a hydrodynamic diameter average and polydispersity index of 118.0 nm ± 0.8 nm and 0.104 nm ± 0.009 nm, respectively (Figure 6). The zeta potential (DLS) was 31.7 mV ± 0.4 mV (Figure 7), suggesting that predominant ending groups on the surface are the metal (inorganic node) units.

### Synthesis and Characterization of PCN-222@PEG-PO₃

mPEG-PO₃ was prepared from a base polymer of poly(ethylene glycol) methyl ether (Mn = 5000 g/mol) in 71% overall yield.

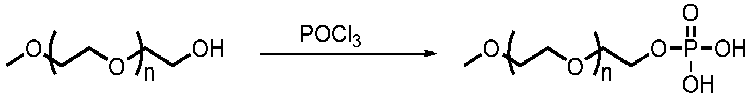

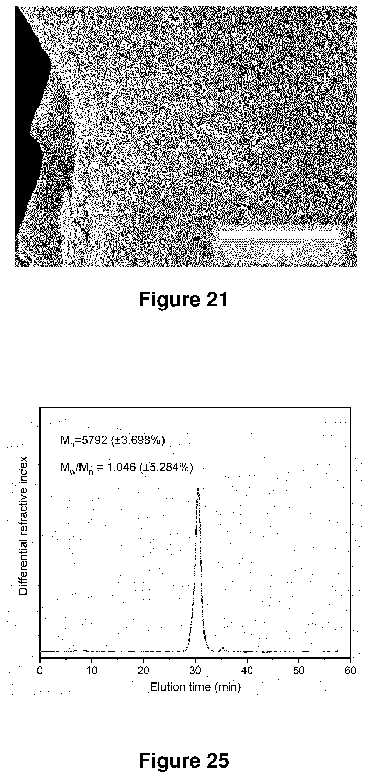

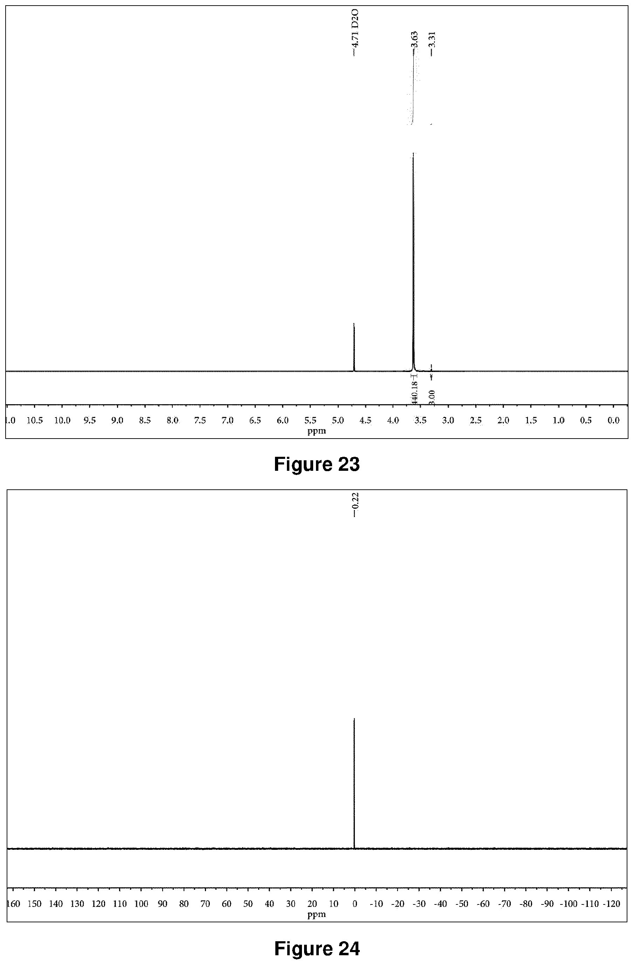

Nuclear magnetic resonance (NMR) and gel permeation chromatography (GPC) spectra confirmed the formation and purity of mPEG-PO₃ (Figures 23, 24 and 25). Only one singlet was observed in ³¹P NMR (Figure 24), indicating that either one or two PEG chains is/are attached to the phosphate group, whereas, Mₙ obtained from GPC (Figure 25) further demonstrated the presence of one PEG chain.

### Procedure for PEGylation of PCN-222 (PCN-222@PEG-PO₃)

mPEG-PO₃ solution (10 mL, 25 mg/mL in H₂O) was added into the water suspension of PCN-222 (5 mL, 10 mg/mL). After stirring at room temperature for 16 h, the reaction mixture was then dialyzed (molecular weight cutoff, 12,000 g/mol -14,000 g/mol) against pure deionized water for 12 h to remove unreacted reagents. The deionized water was decanted and replenished with the fresh one (20 mL, 80 mM) every 4 h. The obtained suspension of PCN-222@PEG-PO₃ was then lyophilised using a Telstar LyoQuest benchtop freeze dryer (0.008 mBar, -70 °C) to give a brown product with relatively low density, or collected by centrifugation (18,000 rpm, 35 min), washed with ethanol and dried under the air, giving a relatively high density and dark coloured product.

Note that the final PEG loading in the PCN-222@PEG-PO₃ nanoparticles was determined by the ratio of phosphate (mPEG-PO₃) to zirconium (PCN-222), as shown in Table 1 below.

The methods for PEGylation of UiO-66@PEG-PO₃, MOF-808@PEG-PO₃, NU-901@PEG-PO₃ and PCN-128@PEG-PO₃ were substantially the same as that of PCN-222@PEG-PO₃.

### Drying PCN-222@PEG-PO₃

The method used to dry the *PCN-222@PEG-PO₃* was shown to affect the morphology of the resulting dry powder.

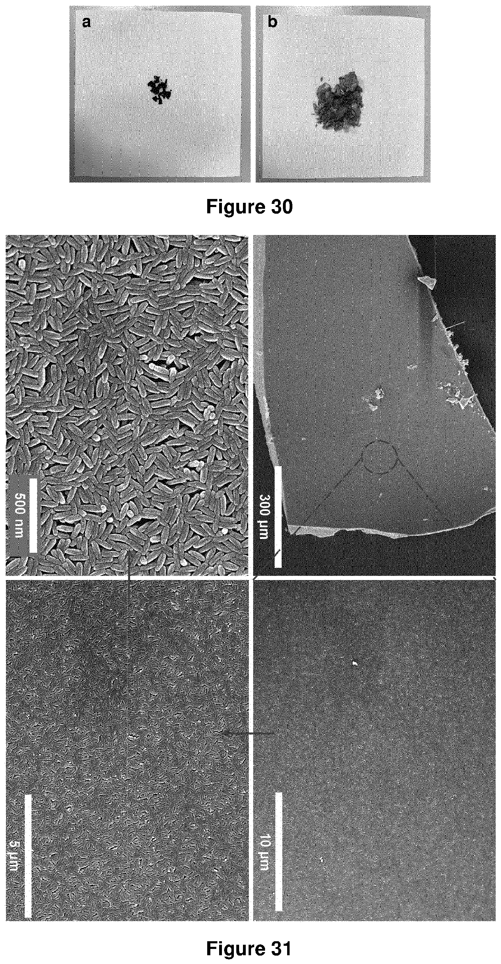

When the above described final product water suspension was spun down, exchanged with ethanol and allowed to dry in ambient conditions (room temperature and pressure), a relatively dense and deep-dark sample was obtained (Figure 30a).

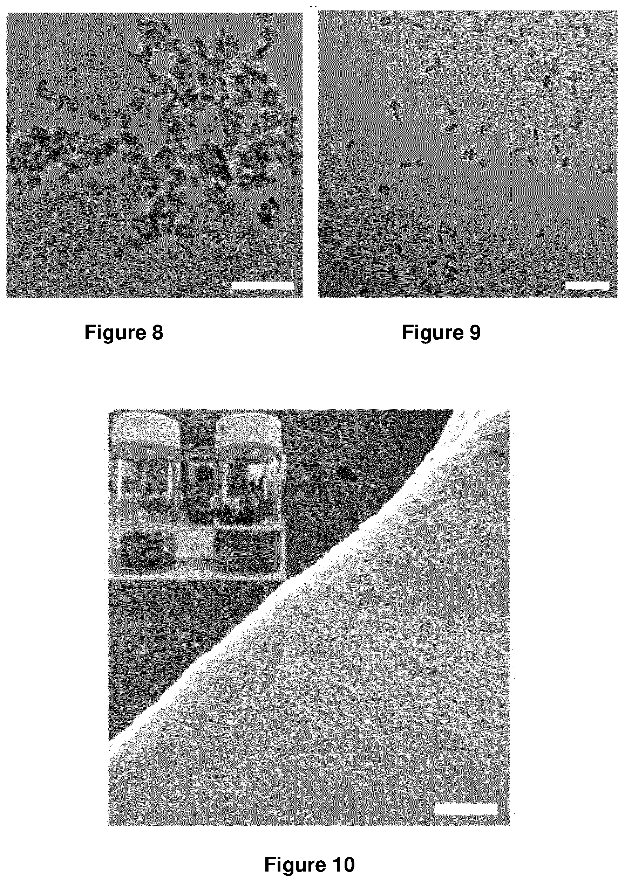

In contrast, when the suspension was lyophilised, a brown, relatively low-density material was obtained (inset of Figure 10 and Figure 30b).

It was found that, while the air-dried material was largely aggregated, the lyophilised sample can be easily re-dispersed by sonication into a pink suspension (Figure 10, inset). A characteristic Tyndall effect was seen by passing a red laser beam, revealing the colloidal nature of the re-dispersed suspension.

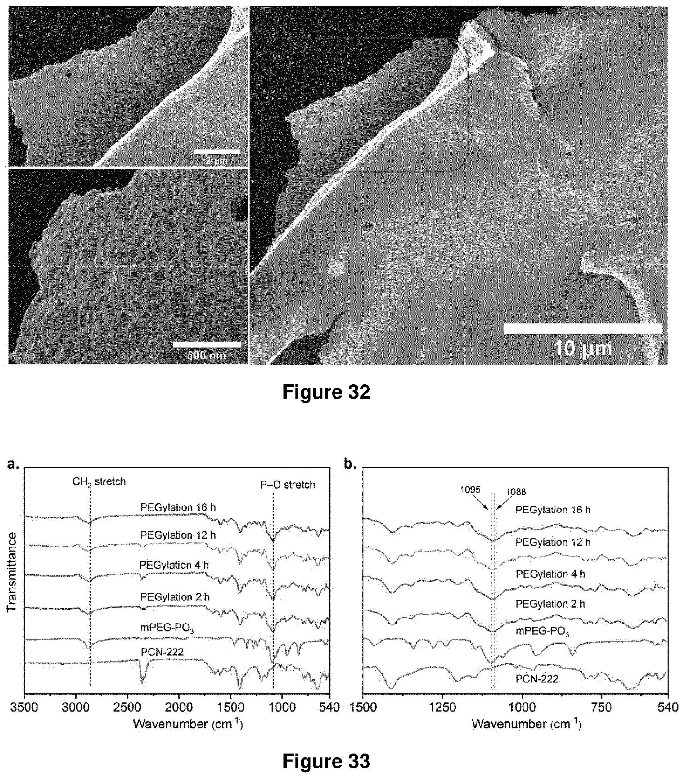

Scanning electron microscopy (SEM) for the dried samples was performed to visualise the presence of mPEG-PO₃ in PCN-222@PEG-PO₃ (Figure 31 and 32). The SEM images clearly indicated the close packing and uniform dispersion of PCN-222@PEG-PO₃ and their rod-shaped morphology.

NanoMOFs tightly embedded within the matrix in the lyophilised PCN-222@PEG-PO₃ were clearly observed. Without being bound by theory, it is believed that the matrix is formed by the surface-attached mPEG-PO₃ molecules (Figure 7 and Figure 32), which can act as a particle partition, thus forming the free-standing PCN-222@PEG-PO₃ particles.

In contrast, SEM of air-dried PCN-222@PEG-PO₃ reveals monodispersed particles randomly packed into centimetre-sized three-dimensional superstructures, where the matrix observed in lyophilised samples is not present. This suggests that in the air-dried samples, the mPEG-PO₃ molecules closely adhere around the particles. In addition, the surface of the nanoMOFs in the air-dried PCN-222@PEG-PO₃ is rough compared to the parent PCN-222, because the surface features of air-dried PCN-222@PEG-PO₃ become dominated by the bulk of the capping polymers, which form nano-papillae on the surface, thus making the surface relatively rough.

### Incorporation of mPEG-PO₃ in PCN-222@PEG-PO₃

Table 1 shows that the amount of incorporated mPEG-PO₃ in PCN-222@PEG-PO₃ is 32.9 % by weight of the coated particles, measured using inductively coupled plasma-optical emission spectroscopy (ICP-OES) by measuring the ratio of P to Zr.

**Table 1. ICP-OES analysis of MOF@PEG-PO₃**

| Sample | P (mg/L) | Zr (mg/L) | PEG loading (wt%) |
|---|---|---|---|
| PEGylation 2 h | 0.046 | 5.114 | 27.8 |
| PEGylation 4 h | 0.058 | 4.972 | 33.3 |
| PEGylation 12 h | 0.071 | 5.979 | 33.6 |
| PEGylation 16 h | 0.057 | 4.926 | 32.9 |
| UiO-66@PEG-PO3 | 0.038 | 3.939 | 37.7 |
| MOF-808@PEG-PO3 | 0.044 | 4.605 | 38.5 |
| NU-901@PEG-PO3 | 0.042 | 3.700 | 30.6 |
| PCN-128@PEG-PO3 | 0.057 | 3.241 | 34.1 |

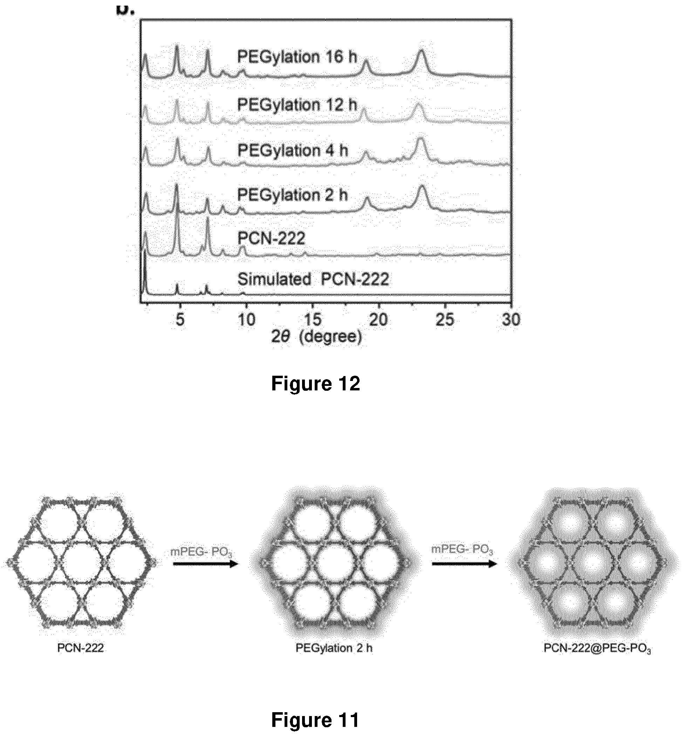

The material was characterised to demonstrate PEGylation of PCN-222. PXRD of PCN-222@PEG-PO₃ correlated well with the parent and simulated patterns, which confirmed the crystallinity of PCN-222 after PEGylation, while the presence of two new peaks centring at 2θ = 19.0° and 23.2° are due to the presence of high molecular weight and semi-crystalline PEG in PCN-222@PEG-PO₃ (Figure 12).

Fourier-transform infrared (FT-IR) spectra provided further evidence for the existence of mPEG-PO₃.The appearance of two new bands at 2866 cm⁻¹ and 1088 cm⁻¹ can be attributed to the stretching vibration of C-H and P-O, respectively, from mPEG-PO₃ molecules (Figure 33a). In particular, a shift was observed in the FT-IR absorption from 1095 cm⁻¹ to 1088 cm⁻¹ for the P-O bonds in the PCN-222@PEG-PO₃ compared to that of mPEG-PO₃ (Figure 33b), indicating the presence of interaction between the Zr₆ cluster and the phosphate group, as well as the fact that all phosphate groups of incorporated mPEG-PO₃ are participating in the coordination and not simply adsorbed on the external surface.

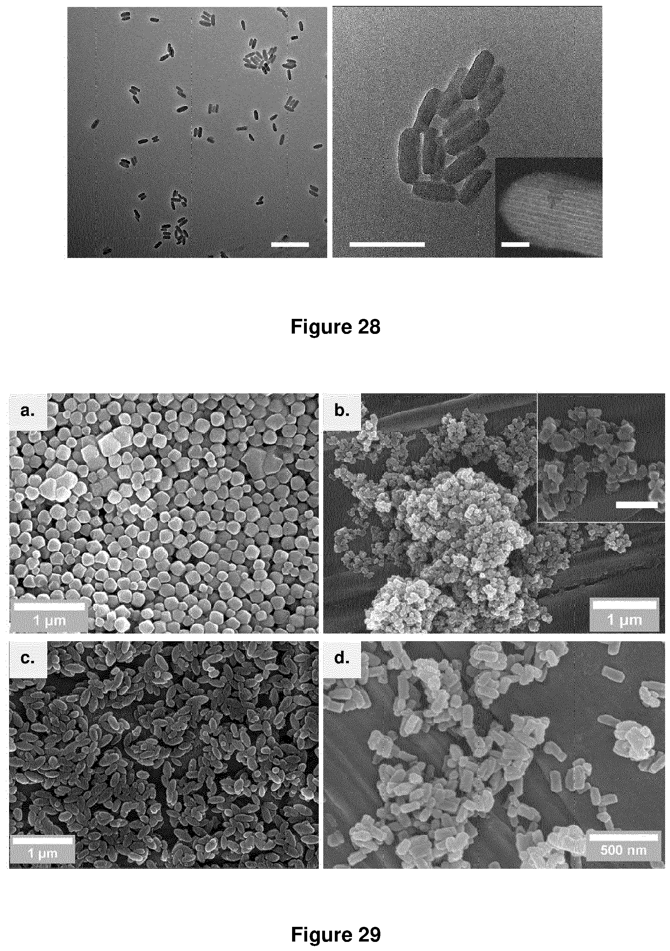

TEM imaging of drop casted PCN-222@PEG-PO₃ clearly shows the well-preserved morphology and generally monodisperse particles of the parent MOF (Figures 8 and 9 for PCN-222 and PCN-222@PEG-PO₃, respectively). Moreover, HAADF-STEM imaging demonstrates the preservation of highly ordered mesopores after PEGylation (Figure 28).

DLS was also employed to investigate the effect of PEGylation on the dispersity of PCN-222. PEGylation of PCN-222 resulted in particles with a particle diameter of 129.7 nm ± 0.9 nm, which is slightly larger than that of the parent PCN-222 (Figure 6). At the same time, the polydispersity index was reduced, 0.076 nm ± 0.004 nm. Without being bound by theory, this can be explained by the PEGylation process dispersing small aggregates in parent PCN-222.

Figure 7 shows that the zeta potential becomes negative, from 31.7 mV ± 0.4 mV for the parent PCN-222 to -43.9 mV ± 1.2 mV for PCN-222@PEG-PO₃, attributed to the attachment of terminal phosphate groups on the external surface of the PCN-222sample.

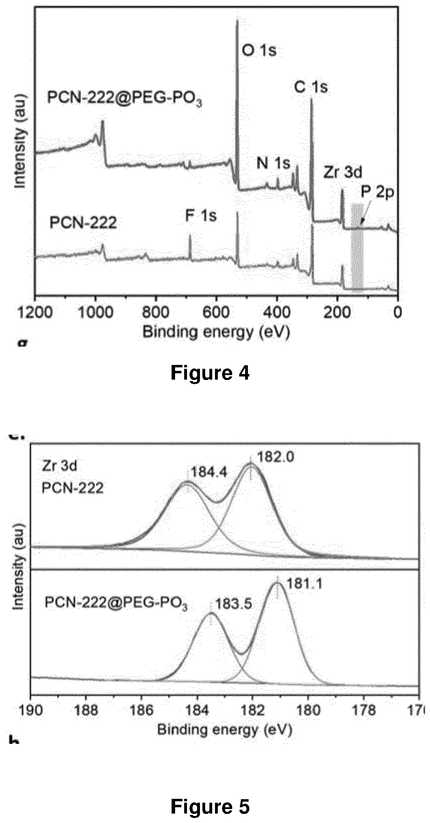

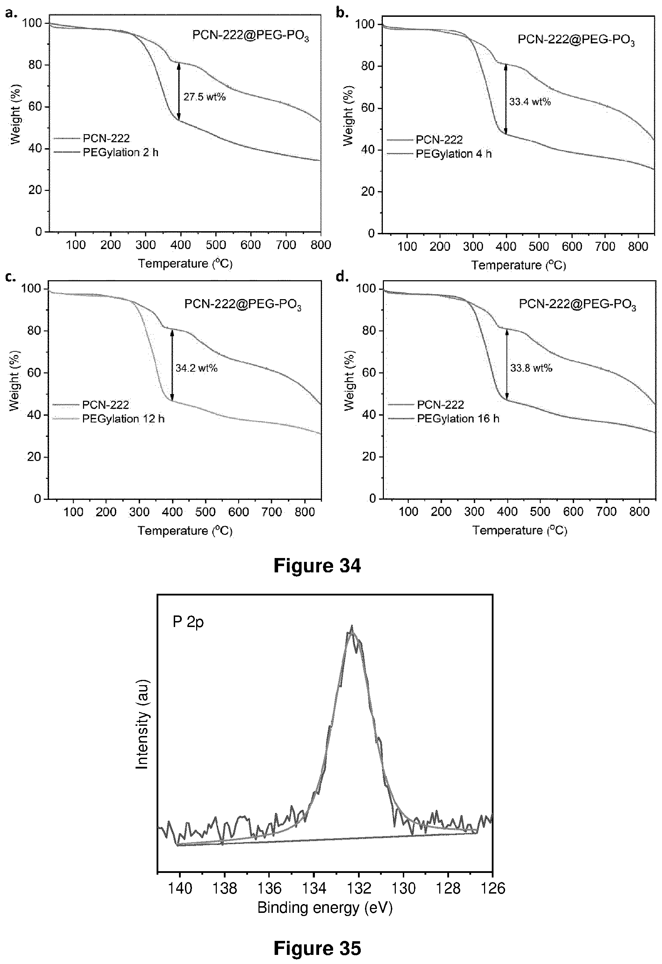

³¹P solid-state nuclear magnetic resonance (SSNMR) and X-ray photoelectron (XPS) spectra were performed to study the interaction between mPEG-PO₃ and framework. Figure 3 shows the chemical shifts of phosphorus resonance in solid-state free mPEG-PO₃ and PCN-222@PEG-PO₃. Free mPEG-PO₃ features a sharp peak at around -0.30 ppm; after PEGylation of PCN-222, the peak broadens and upshifts to -8.39 ppm. The shift of phosphorus resonance indicates an interaction between the phosphate group of the mPEG-PO₃ and the MOF. The XPS survey and high-resolution of P 2p spectra confirm the existence of phosphorous in PCN-222@PEG-PO₃ (Figure 4 and Figure 35), which belongs to the phosphorous of incorporated mPEG-PO₃.

Furthermore, in the high-resolution Zr 3d spectra, two main peaks at around 184.4 eV and 182.0 eV, ascribed to the 3d_{3/2} and 3d_{5/2} of Zr(IV) in parent PCN-222, downshift to 183.5 eV and 181.1 eV, respectively (Figure 5).

Without being bound by theory, it is believed that the shift of binding energy to lower energies may be due to the replacement of the TFA coordinated on Zr-O clusters in PCN-222 with mPEG-PO₃ in PCN-222@PEG-PO₃.Compared to the trifluoroacetate group of TFA, the phosphate group of mPEG-PO₃ is less electronegative. This bonding results in a lower loss of electron density at Zr, which in turn decreases its 3d electrons binding energies. Figure 4 shows that the F 1s peak of parent PCN-222 at 688.2 eV is significantly weakened after the PEGylation, suggesting the amount of TFA bonded decreases.

Taken together, the shift of chemical resonances and binding energies after the PEGylation demonstrates the formation of the Zr-O-P coordination bonds.

### Impact of mPEG-PO₃ on Internal Porosity

N₂ uptake experiments were then conducted at 77 K to investigate the impact of the incorporation of mPEG-PO₃ into the internal porosity of PCN-222. As shown in Figure 13, PCN-222 and PCN-222@PEG-PO₃ adsorb 526 cm³g⁻¹ and 129 cm³g⁻¹ N₂ at P/P₀ = 0.8, respectively.

Assuming that the included mPEG-PO₃ locates at the external surface of nanoPCN-222, the ideal N₂ uptake at P/P₀ = 0.8 would be 353 cm³g⁻¹ after subtracting the amount of mPEG-PO₃ included (Table 2), the actual value (129 cm³g⁻¹ N₂) obtained here accounts for 36.5 % of the ideal N₂ uptake.

Without being bound by theory, it is believed that the discrepancy between the ideal value and the experimental value may be due to the infiltration of mPEG-PO₃ molecules into the internal porosity of nanoPCN-222 after performing PEGylation for 16 h. Moreover, pore size distribution (PSD) obtained using the Non-Local Density Functional Theory (NLDFT) method also revealed the decreased porosity (Figure 14), further demonstrating that the present invention modifies the MOF nanoparticle surface with a partial sacrifice of its porosity.

### Time-Dependency Studies

Time-dependent studies were performed by quenching the PEGylation of PCN-222 at different reaction times and analysing the intermediates with a number of ex situ spectroscopic and microscopic characterisations. These obtained PEGylated PCN-222 were named as PEGylation x h, where x is the reaction time (x = 2, 4, 12, 16).

### Synthesis for Time-dependency study

To four identical vials containing 5 mL water suspension of 120 nm-sized PCN-222 (10 mg/mL) were added with 10 mL mPEG-PO₃ solution (25 mg/mL in H₂O), respectively. The mixture was stirred (800 rpm) at room temperature for 2, 4, 12 and 16 h.

At each time point, one sample was quenched by centrifuge to remove the unreacted mPEG-PO₃, followed by putting it into a dialysis tube (molecular weight cutoff, 12,000 g/mol - 14,000 g/mol), and dialyzed against pure deionised water for 12 h. The deionized water was decanted and replenished with a fresh one (20 mL, 80 mM) every 4 h.

The obtained suspension was then lyophilised using a Telstar LyoQuest benchtop freeze dryer (0.008 mBar, -70 °C) to give the desired products, named PEGylation x h (x is the reaction time), and used for further characterizations.

### Results of Time-dependency Study

Figures 12 and 33a show no significant change in their PXRD patterns and FT-IR spectra throughout the whole PEGylation process. ICP-OES quantifies the amount of incorporated mPEG-PO₃ at different PEGylation times by measuring the ratio of Zr to P (Table 1). The results show rapid incorporation of mPEG-PO₃, the loading of mPEG-PO₃ is 27.8 wt% after 2 h. With increased reaction time, the values plateau at around 33 wt%.

Additionally, thermogravimetric analysis (TGA) profiles show a similar trend (Figure 34). The encapsulated amount of mPEG-PO₃ was calculated based on the difference between the TGA curve of the first plateau (376-452 °C).

N₂ sorption properties were examined to investigate the location of mPEG-PO₃ at different reaction times. After 2 h, the N₂ uptake at P/P₀ = 0.8 decreases to 348 cm³g⁻¹ (Figure 13), accounting for 91.6% of the ideal N₂ uptake (Table 2). Without being bound by theory, it is believed that this is due to the fact that PEGylation mainly occurs at the surface with a small amount of mPEG-PO₃ molecules or the PEG chains of surface-bonded mPEG-PO₃ infiltrating into the porosity.

**Table 2. N₂ uptake at 77 K**

| Sample | Actual N₂ uptake at P/P₀ = 0.8 (cm³g⁻¹, STP) | Ideal N₂ uptake at P/P₀ = 0.8 (cm³g⁻¹ , STP) | Actual uptake/Ideal uptake (%) |
|---|---|---|---|
| PCN-222 | 526 | -- | -- |
| PEGylation 2 h | 348 | 380 | 91.6 |
| PEGylation 4 h | 124 | 351 | 35.3 |
| PEGylation 12 h | 121 | 349 | 34.7 |
| PEGylation 16 h | 129 | 353 | 36.5 |

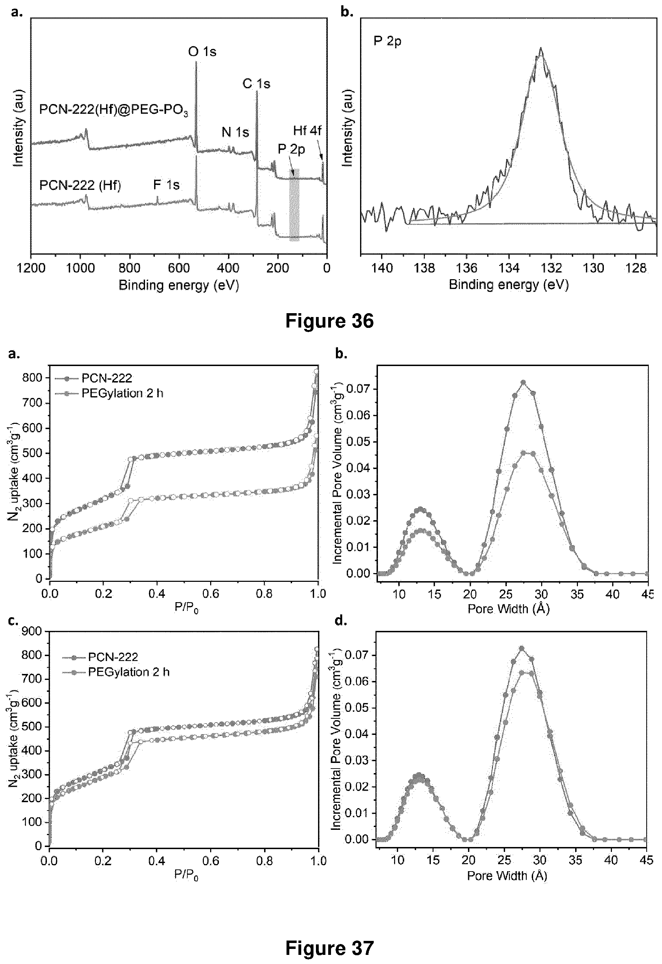

The effect of mPEG-PO₃ over porosity after 2 h can be more easily observed after subtracting the amount of the attached mPEG-PO₃.As shown in Figure 37, N₂ isotherms and PSD results indicate only a small decrease in its N₂ isotherm and PSD. Again without being bound by theory, these results suggest that PEGylation mainly occurs on the surface at the early stage. Afterwards, with continuous PEGylation for 4 h, the amount of mPEG-PO₃ increases up to 33.3 wt% (Table 1), the corresponding N₂ uptakes at P/P₀ = 0.8 decreases to 124 cm³g⁻¹, which accounts for 35.3% of the ideal N₂ uptake (Table 2), and these values become constant even at prolonged reaction times of 12 h and 16 h (Figure 13) indicating that PEGylation reaches equilibrium and mPEG-PO₃ molecules enter the pore of the particles. Without being bound by theory, it is believed that the excess mPEG-PO₃ molecules start to infiltrate into the channel after substantially fully occupying the available binding sites at the external surface.

Taken together, the above results demonstrate a two-step PEGylation process for PCN-222 with mPEG-PO₃ is proposed. Without being bound by theory, the PEGylation firstly takes place at the external surface due to the electrostatic interaction between positive-charged PCN-222 and negative-charged mPEG-PO₃.After occupying the available binding sites at the surface, unreacted mPEG-PO₃ molecules start to enter the internal channel of PCN-222, thus reducing the porosity (Figure 11).

### Elemental Mapping

Elemental mapping was conducted, employing energy-dispersive X-ray (EDX) spectroscopy in a STEM. However, because the La line for Zr is 2.042 keV and the Ka line for P is 2.012 keV, the energy window created for P would overlap with that of Zr leading to misidentification of elements.

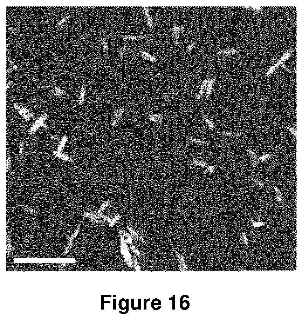

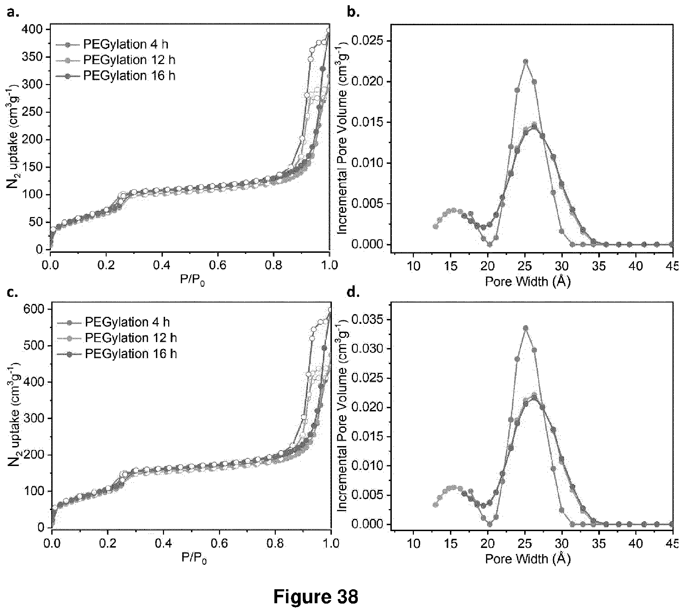

This issue was addressed by forming an alternative polymer-coated nanoparticle MOF by replacing Zr-based PCN-222 with Hf-based PCN-222, (denoted as PCN-222 (Hf)). PXRD and N₂ isotherm confirmed the crystallinity and porosity of PCN-222 (Hf) (Figures 15, 38 and 40), STEM imaging and XPS spectrum clearly showed the rod morphology of PCN-222 (Hf) and the presence of Hf (Figure 16 and Figure 36).

Under identical PEGylation conditions to that of Zr-based PCN-222, the ex situ STEM-EDX of PCN-222 (Hf) after 2 h, 4 h, 12 h and 16 h was obtained, which mapped out the distribution of P and Hf. After 2 h, the signal of Hf is evenly distributed throughout the whole particle, whereas the P signal mainly originated from the edges. This provided further evidence to demonstrate that mPEG-PO₃ molecules mainly graft the surface at the early stage. With the progress of reaction time from 4 h to 16 h, P signals are uniformly distributed throughout the whole particles.

Additionally, EDX line scans for the PCN-222 (Hf) after PEGylation of 16 h were also performed, these demonstrated the homogeneous distribution of Hf and P along the single particle. The results observed here are very consistent with TGA, N₂ uptake and ICP-OES as mentioned above, and confirm that the PEGylation of PCN-222 and PCN-222 (Hf) nanoparticles with mPEG-PO₃ proceed in a two-step fashion (Figure 11).

### Versatility of the Phosphate Coordination PEGylation Method

The above PEGylation method was extended to four additional Zr-based MOFs, including UiO-66, MOF-808, NU-901 and PCN-128 (Figure 29). The related lyophilised PEGylated MOFs were successfully obtained, denoted as MOF@PEG-PO₃, using the same nomenclature as for PCN-222.

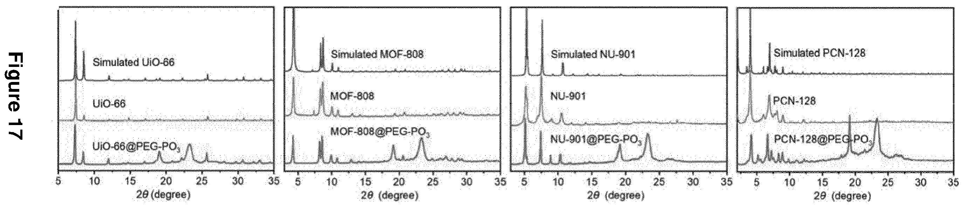

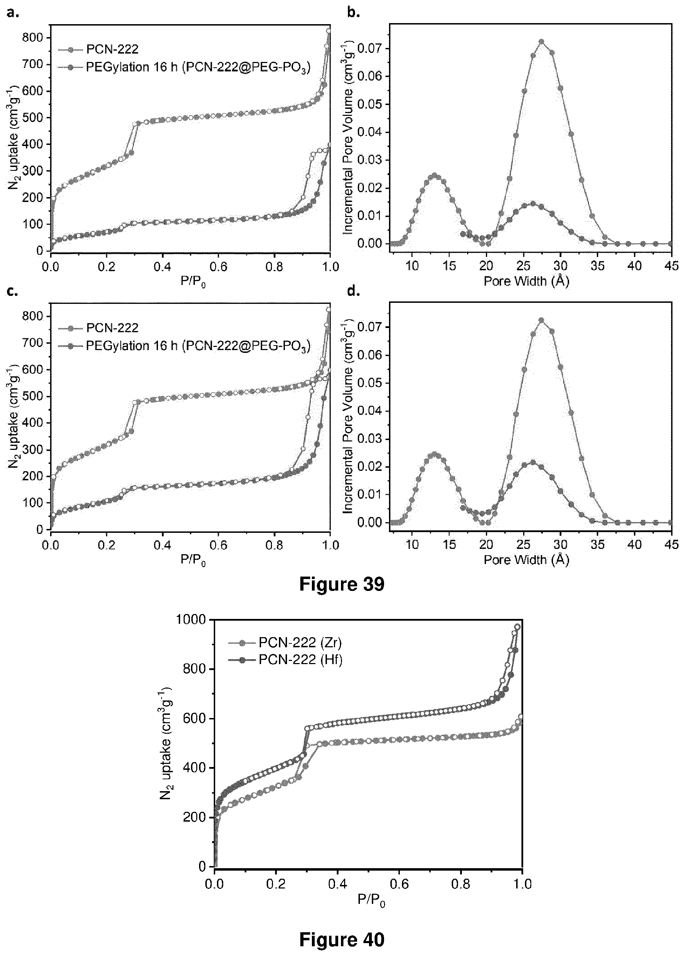

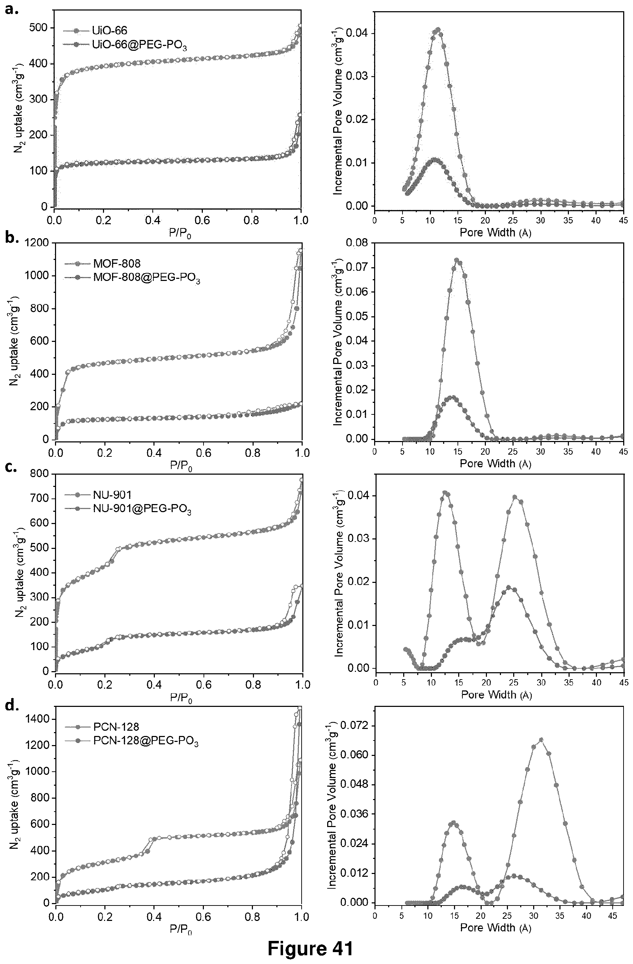

A pronounced Tyndall effect was recorded by passing a red laser beam through these MOF@PEG-PO₃. Figure 17 shows the retaining of crystallinities and the presence of semicrystalline PEG after PEGylation, proven by PXRD tests. N₂ isotherms and PSD show that porosities are all partially blocked by the infiltrated mPEG-PO₃, similar to that of PCN-222@PEG-PO₃ (Figures 39 and 41).

The loading of incorporated mPEG-PO₃ molecules evaluated by ICP-OES are 37.7 wt%, 38.5 wt%, 30.6 wt% and 34.1 wt% for UiO-66@PEG-PO₃, MOF-808@PEG-PO₃, NU-901@PEG-PO₃ and PCN-128@PEG-PO₃, respectively (Table 1).

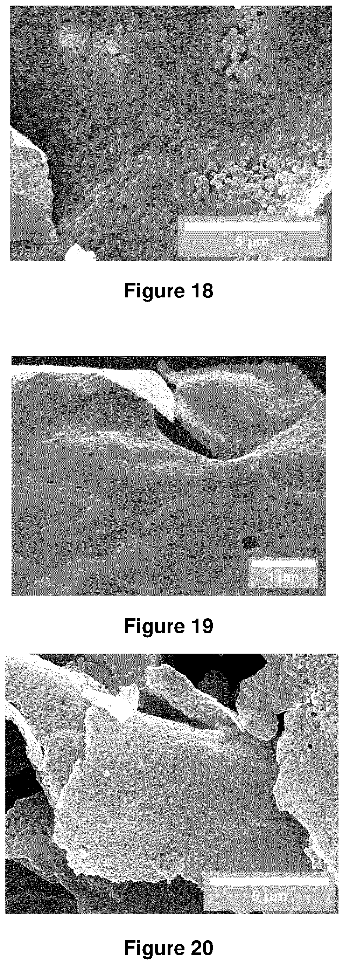

All the lyophilised MOF@PEG-PO₃ have relatively low densities, SEM images of the lyophilised MOF@PEG-PO₃ exhibit the uniform distribution of particles within the matrix formed by incorporated mPEG-PO₃ molecules (Figures 18, 19, 20 and 21).

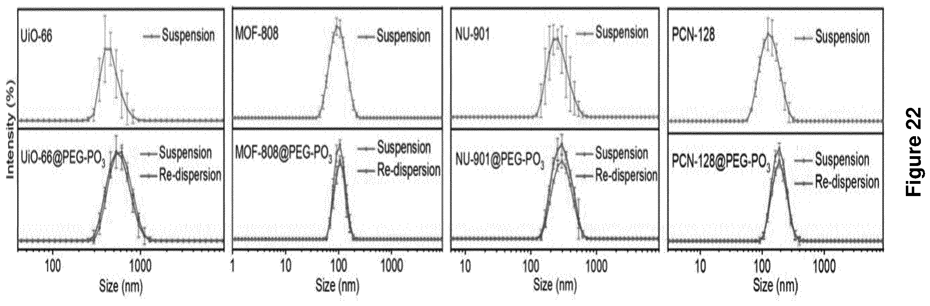

Importantly, all of the MOF@PEG-PO₃ nanoparticles could be re-dispersed in water easily after mild sonication. There was no significant difference in their hydrodynamic diameters compared with the suspensions before the lyophilisation (Figure 22).

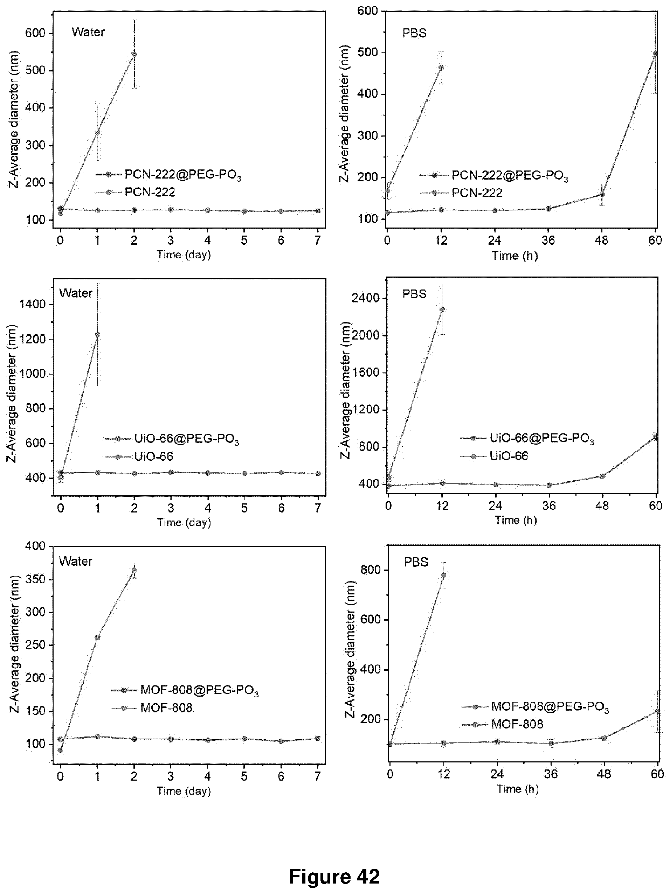

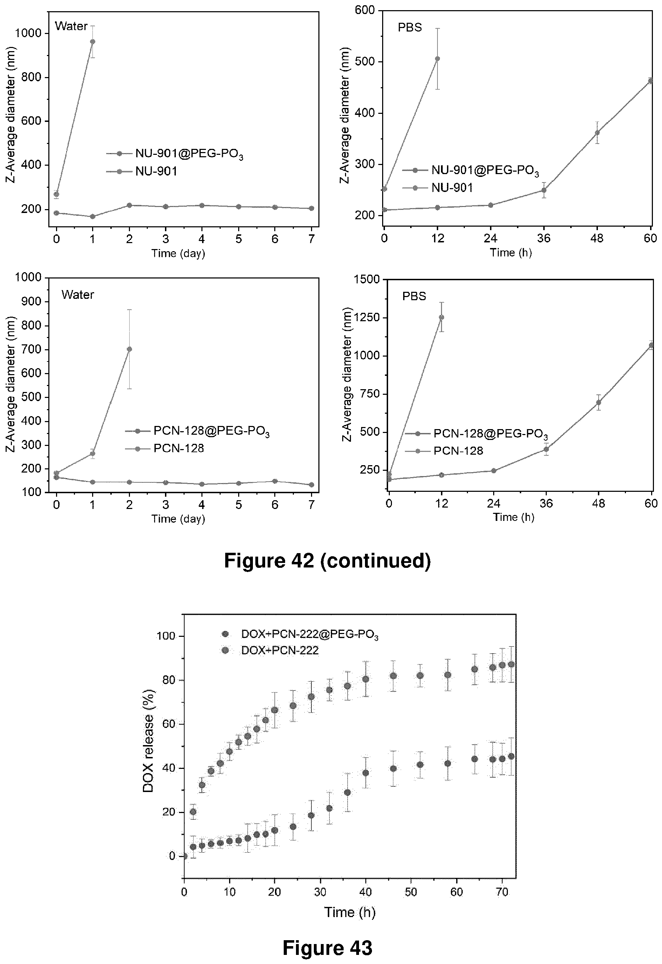

The colloidal stability of these MOF@PEG-PO₃ in water and phosphate buffered saline (PBS; pH = 7.4) was evaluated. Figures 41 and 42 showed that the suspensions of PEGylated MOFs in water can be stable up to 7 days without significant changes in their hydrodynamic sizes, outperforming the bare MOFs, which aggregate seriously over time. In the case of PBS (pH = 7.4), MOF@PEG-PO₃ can remain their hydrodynamic sizes for 36 h.

In contrast, the bare MOF nanoparticles precipitated in a very short time, for example, within one day.

These results confirm that the polymer coating method disclosed herein can act as a versatile strategy to not only slowdown the aggregation in water and biological media, but also provide an alternative for the long-term storage of MOF-based drug carriers.

### Controlled Release Study - DOX@PCN-222@PEG-PO₃

The polymer-coated MOF nanoparticles of the present invention can be used to encapsulate an active ingredient as described herein above. The release profile of encapsulated active ingredient from a polymer-coated MOF nanoparticle is particularly advantageous when compared to the active ingredient release profile of uncoated MOF nanoparticles.

Note that the nomenclature used for MOF nanoparticles encapsulating an active ingredient is active@MOF for an uncoated MOF nanoparticle and active@MOF@polymer for a polymer-coated MOF nanoparticle. For example, doxorubicin (DOX) encapsulated in PCN-222 or PCN-222@PEG-PO₃ is denoted as DOX@PCN-222 and DOX@PCN-222@PEG-PO₃, respectively.

### Preparation of DOX@PCN-222

20 mg of PCN-222 was soaked in a 15 mg/mL doxorubicin (DOX) in deionised H₂O solution for 24 h at room temperature. After that, the obtained DOX-loaded PCN-222 (DOX@PCN-222) was collected by 20 min of centrifugation at 14000 rpm and rinsed with deionised water to remove the surface physiosorbed DOX molecules. The obtained DOX@PCN-222 was then dried in a vacuum oven at room temperature for 24 h.

### Preparation of DOX@PCN-222@PEG-PO₃

20 mg of PCN-222 was soaked in a 15 mg/mL DOX in deionised H₂O solution for 24 h at room temperature. After that, the obtained DOX-loaded PCN-222 (DOX@PCN-222) was collected by 20 min of centrifugation at 14000 rpm, the 5 equivalent (mass ratio) mPEG-PO₃ was added and stirred for another 16 h. The final sample was collected by 20 min of centrifugation at 14000 rpm and rinsed with deionised water to remove the surface physiosorbed DOX molecules. The obtained DOX@PCN-222@PEG-PO₃ was then dried in a Telstar LyoQuest benchtop freeze dryer (0.008 mBar, -70 °C).

To determine the extent of drug (DOX) loading, the supernatant of the centrifuged solution was collected and subjected to UV-vis spectrometry at 482 nm. To specify the concentration of the drug, the recorded absorption intensity was compared with a calibration curve that was plotted for free DOX.

### Controlled-release Testing

8 mg DOX loaded sample was kept in a dialysis bag (molecular cutoff: 12,000-14,000 Da). The dialysis bag was immersed in 30 mL deionised water and stirred at room temperature. 3 mL of the supernatant was taken out and analysed by UV-vis spectrometer at 482 nm to determine the released DOX concentration every 2 h. After completion of each measurement, the removed solvent was added to the mixture to keep its volume fixed.

As shown in Figure 43, the release of DOX from uncoated MOF nanoparticles (PCN-222) is significantly faster than the release of DOX from polymer-coated MOF nanoparticles (PCN-222@PEG-PO₃). Approximately 40% DOX release was recorded after 6 hours from DOX@PCN-222. Whereas, approximately 40% DOX release was recorded after 40 hours from DOX@PCN-222@PEG-PO₃.

### Controlled Release Study - Further exemplary DOX@MOF@PEG-PO3

A further study was performed to demonstrate the advantageous controlled, sustained release of an active ingredient, in particular an active pharmaceutical ingredient, from the polymer-coated metal-organic framework nanoparticles of the present invention.

### MOF Loading with DOX

*DOX@MOF:* An aqueous as-synthesized nanoMOF (5 mg/mL, 6 mL) suspension was added to an aqueous DOX solution (15 mg/mL, 4 mL), the mixture was then stirred for 48 hours at room temperature. Afterwards, the DOX loaded nanoMOFs were collected by centrifugation (15,000 rpm, 35 min) and washed with water for 3 times. The obtained DOX@MOF was dried under vacuum or redispersed in water for the future use. The unloaded DOX was combined and diluted to a final volume of 500 mL for DOX loading determination.

DOX@MOF@PEG-PO₃; The aqueous as-synthesized nanoMOFs (5 mg/mL, 6 mL) suspension was added to an aqueous DOX solution (15 mg/mL, 4 mL), the mixture was then stirred for 48 hours at room temperature. Afterwards, the aqueous mPEG-PO₃ solution (6 mL, 25 mg/mL) was added directly, the resulting mixture was stirred for another 16 h. The reaction mixture was then centrifuged (15,000 rpm, 35 min) to remove the unreacted mPEG-PO₃ and unloaded DOX, and redispersed in water, followed by dialysis (MWCO, 12,000-14,000) against water for 12 h. The water was decanted and replenished with fresh water every 4 h. The final suspension was lyophilized (0.008 mBar, -70 °C) or dried under vacuum. The unloaded DOX was combined and diluted to a final volume of 500 mL for DOX loading determination.

The absorbance was determined by UV-Vis spectroscopy (maximum absorbance at 486 nm). The concentration was calculated by comparing the UV-Vis absorbance with a calibration curve.

Drug loading (wt %) = (mass of drug used - mass of unloaded drug) / mass of MOFs x 100.

### Drug release

10 mg DOX@MOF and DOX@MOF@PEG-PO₃ were suspended in 2 mL PBS (pH = 7.4) and placed in dialysis bags (MWCO; 1,2000 - 1,4000) dispersed with 2 mL PBS (pH = 7.4), dialyzing against PBS (pH = 7.4) (28 mL) under magnetic stirring at room temperature. 1 mL of PBS (pH = 7.4) was taken out and replaced with fresh one at specific time intervals. The drug released was analysed by a fluorescence spectroscopy through comparing with their calibration curves mission maximum at 595 nm when excited at 486 nm.

### Drug Loading Results

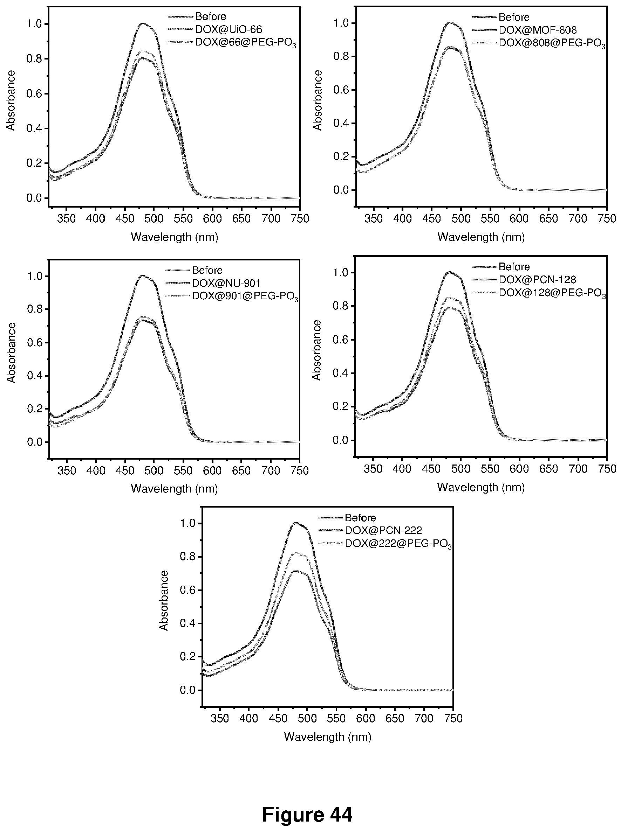

UV-Vis spectroscopy verified 21.3 wt%, 16.8 wt%, 28.5 wt%, 22.1 wt% and 30.2 wt% loadings of DOX were obtained for the bare UiO-66, MOF-808, NU-901, PCN-128 and PCN-222. Only a slight decrease to 17.8 wt%, 15.5 wt%, 25.3 wt%, 15.8 wt% and 18.4 wt%, respectively, was observed with PEGylation (Figure 44). Without being bound by theory, it is believed that this was due to a small amount of loaded DOX being displaced by the infiltration of mPEG-PO₃.

### Drug Release Results

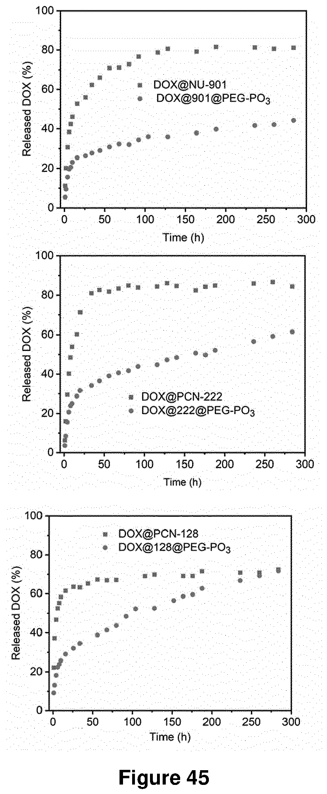

For both DOX@MOF and DOX@MOF@PEG-PO3, cumulative release was monitored for up to 200 h in PBS (pH = 7.4). Figure 45 shows the release kinetics of each of NU-901, PCN-128 and PCN-222.

For NU-901, PCN-128 and PCN-222, the release profiles of DOX@MOF@PEG-PO3 drastically decreased release profiles were obtained for the PEGylated samples, compared to that of the bare (uncoated) nanoMOFs, with less that -20 wt% of the encapsulated DOX being released in the first 6 h. In contrast, the bare nanoMOFs released 38.4 wt%, 40.1 wt% and 52.5 wt% of the DOX respectively.

These results demonstrated slower and more sustained release for polymer-coated MOF nanoparticles. This may be particularly advantageous for sustained active ingredient delivery, particularly sustained pharmaceutical delivery. This sustained release may facilitate, amongst other things, reduced pharmaceutical dosage frequency relative to the direct pharmaceutical delivery or pharmaceutical delivery from uncoated MOF nanoparticles.

Without being bound by theory, it is believed that the polymer-coated nanoparticles of the invention may be particularly effective as a consequence of the relatively strong bonding (e.g. phosphate-metal coordination) between the polymer and MOF nanoparticle, avoiding dumping of the polymer in solution and subsequent rapid dispersion of the medicament. By varying the amount and/or species of polymer used the drug release profile may be tuned.

The use of linear polymers, and in particular linear polyethylene glycol and derivatives thereof, have been found to be particularly effective. Without being bound by theory it is believed that this is because the linear nature of the polymers helps keep the nanoparticles far enough apart that they can be re-dispersed.

Advantageously, therefore, the lyophilised polymer-coated pharmaceutical-containing MOF nanoparticles may provide improved storage lifetime, facilitate redispersion with reduced agglomeration, and provide tuneable, sustained release.

Every document cited herein, including any cross-referenced or related patent or application, is hereby incorporated by reference in its entirety unless expressly excluded or otherwise limited.

It will be appreciated that various modifications may be made to the embodiments shown without departing from the spirit and scope of the invention as defined by the accompanying claims.

## Claims

1. A dry powder pharmaceutical composition comprising one or more lyophilised polymer-coated metal-organic framework (MOF) nanoparticles,
each nanoparticle comprising a MOF comprising a plurality of metal ions and a plurality of organic ligands and having a particle size of from about 25 nm to about 450 nm;
wherein the polymer is attached to one or more metal ions,
and wherein at least one active pharmaceutical ingredient is located on and/or within one or more of the lyophilised polymer-coated MOF nanoparticles, preferably wherein the polymer is attached to one or more metal ions of each nanoparticle by phosphate-metal coordination.

2. A pharmaceutical composition comprising the dry powder pharmaceutical composition of any of claim 1 mixed into a liquid; preferably wherein the liquid is aqueous; preferably wherein the composition further comprises a pharmaceutically acceptable excipient; preferably wherein the excipient is selected from the group consisting of solvents, co-solvents, buffers, stabilisers, antioxidants, preservatives, chelating agents, emulsifiers, flavourings, lubricants, suspending agents, tonicity adjusting agents, surfactants, solubilisers, suspending aids, dispersion agents, humectants, thickeners, colouring agent, wetting agent, anti-foaming agent, viscosity modifier, sweeteners and combinations thereof.

3. A composition comprising one or more polymer-coated metal-organic framework (MOF) nanoparticles, each nanoparticle comprising a MOF comprising a plurality of metal ions and a plurality of organic ligands; wherein the polymer is attached to one or more metal ions of each nanoparticle by phosphate-metal coordination; and wherein the particle size of the one or more polymer-coated MOF nanoparticles is from about 25 nm to about 450 nm.

4. The composition according to claim 3, wherein an active ingredient is encapsulated within and/or located on the one or more polymer-coated MOF nanoparticles, preferably where in the active ingredient is an active pharmaceutical ingredient.

5. A method of producing a dry powder composition comprising one or more polymer-coated metal-organic framework (MOF) nanoparticles, each nanoparticle comprising a MOF comprising a plurality of metal ions and a plurality of organic ligands; wherein the polymer is attached to one or more metal ions; wherein the particle size of the one or more polymer-coated MOF nanoparticles is from about 25 nm to about 450 nm; the method comprising:
a) mixing a plurality of metal ions and a plurality of organic ligands to form a suspension of one or more uncoated MOF nanoparticles,
b) mixing a suspension of the one or more uncoated MOF nanoparticles with the polymer and allowing the polymer to attach to one or more metal ions of the one or more uncoated MOF nanoparticles to provide a suspension of the one or more polymer-coated MOF nanoparticles,
c) optionally increasing the concentration of the polymer-coated MOF nanoparticle suspension by removing the solvent,
d) drying the one or more polymer-coated MOF nanoparticles by lyophilisation.
wherein an active ingredient is optionally encapsulated within and/or located on the one or more polymer-coated MOF nanoparticles, preferably prior to step b), preferably by mixing an active ingredient with a suspension of the one or more uncoated MOF nanoparticles.

6. A method of producing a suspension of polymer-coated MOF nanoparticles, the method comprising the step of taking the dry powder composition produced according to claim 5 and resuspending the composition in a liquid, preferably wherein the liquid is aqueous.

7. A method of producing a composition comprising one or more polymer-coated active ingredient-containing metal-organic framework (MOF) nanoparticles, each nanoparticle comprising a MOF comprising a plurality of metal ions and a plurality of organic ligands and having a particle size from about 25 nm to about 450 nm; wherein polymer is attached to one or more metal ions by phosphate-metal coordination; the method comprising:
a) mixing a plurality of metal ions and a plurality of organic ligands to form a suspension of one or more uncoated MOF nanoparticles,
b) encapsulating an active ingredient within and/or locating an active ingredient on one or more of the uncoated MOF nanoparticles to provide a suspension of one or more active ingredient-containing, uncoated MOF nanoparticles,
c) subsequently mixing a suspension of the one or more active ingredient-containing, uncoated MOF nanoparticles with a phosphate-functionalised polymer and allowing the phosphate-functionalised polymer to coordinate to one or more metal ions of the one or more active ingredient-containing, uncoated MOF nanoparticles to provide a suspension of one or more active ingredient-containing polymer-coated MOF nanoparticles,
d) optionally increasing the concentration of the one or more active ingredient-containing polymer-coated MOF nanoparticle suspension by removing the solvent,
e) optionally drying the one or more polymer-coated active ingredient-containing MOF nanoparticles by lyophilisation, and
f) optionally, subsequently redispersing the lyophilised polymer-coated active-ingredient-containing MOF nanoparticles in an aqueous medium.

8. A method of producing a composition comprising a one or more methoxy polyethylene glycol (mPEG)-coated metal-organic framework (MOF) nanoparticles, each nanoparticle comprising a MOF comprising a plurality of metal ions and a plurality of organic ligands and having a particle size of from about 25 nm to about 450 nm; wherein mPEG is attached to one or more metal ions by phosphate-metal coordination; and wherein the method comprises:
a) mixing a plurality of metal ions and a plurality of organic ligands to form a suspension of one or more uncoated MOF nanoparticles,
b) mixing a suspension of the one or more uncoated MOF nanoparticles with a solution of mPEG-PO₃ and allowing mPEG-PO₃ to coordinate to one or more metal ions of the one or more uncoated MOF nanoparticles to provide a suspension of the one or more mPEG-coated MOF nanoparticles,
c) optionally increasing the concentration of the mPEG-coated MOF nanoparticles suspension by removing solvent,
d) optionally drying the one or more mPEG-coated MOF nanoparticles by lyophilisation, and
e) optionally redispersing the lyophilised mPEG-coated MOF nanoparticles, preferably by sonication;
wherein an active ingredient is optionally located on and/or encapsulated within the one or more mPEG-coated MOF nanoparticles, preferably prior to step b), preferably by the step of mixing an active ingredient with a suspension of the one or more uncoated MOF nanoparticles.

9. The composition or method according to any of the preceding claims, wherein the plurality of metal ions consist essentially of a metal ion selected from the group consisting of: zirconium, iron, zinc, and hafnium.

10. The composition or method according to any of claims 1 to 7 or claim 9 when dependent on claims 1 to 7, wherein the polymer comprises a phosphate group, preferably a terminal phosphate group, preferably wherein the polymer is selected from the group consisting of polyethylene glycol, hyaluronic acid, heparin, chitosan, polyvinyl alcohol, polyglutamic acid, and derivatives and copolymers thereof; preferably wherein the polymer comprises polyethylene glycol or a derivative thereof, preferably a phosphate-terminated mPEG, preferably wherein the polymer and/or mPEG-PO₃ is a phosphate-terminated mPEG according to structure A 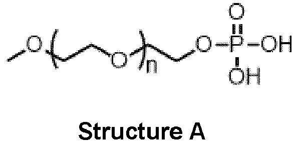 wherein n is from about 40 to about 250

11. The composition or method according to any of the preceding claim, wherein the particle size of the one or more polymer-coated MOF nanoparticles and/or the one or more mPEG-coated MOF nanoparticles is from about 100 nm to about 250 nm, preferably from about 100 nm to about 200 nm.

12. A composition produced according to any of the methods of claims 5 to 8 and/or a composition according to any one of claims 1 to 4 or 9 to 11 wherein the one or more MOF nanoparticles are at least partially amorphous.

13. Use of a phosphate-functionalised polymer to coat one or more MOF nanoparticles; wherein an active ingredient is located within and/or on the one or more polymer-coated MOF nanoparticles.

14. Use of polymer to improve redispersion of a plurality of lyophilised MOF nanoparticles, wherein the polymer is coated onto the MOF nanoparticles prior to lyophilisation.

15. A method of treating a disease or condition by administering a composition according to any one of claims 1 to 4 or 9 to 12 to a patient.
